(19) Europäisches Patentamt
European Patent Office
Office européen des brevets

(11) Numéro de publication: **0 053 072**
**B1**

(12) # FASCICULE DE BREVET EUROPEEN

(45) Date de publication du fascicule du brevet: **08.08.84**

(51) Int. Cl.³: **C 07 D 498/04** // (C07D498/04, 265/00, 205/00)

(21) Numéro de dépôt: **81401830.5**

(22) Date de dépôt: **19.11.81**

(54) **Nouvelles oxacéphalosporines et leur préparation.**

(30) Priorité: **20.11.80 FR 8024639**

(43) Date de publication de la demande: **02.06.82 Bulletin 82/22**

(45) Mention de la délivrance du brevet: **08.08.84 Bulletin 84/32**

(84) Etats contractants désignés: **AT BE CH DE FR GB IT LI LU NL SE**

(56) Documents cités:
**EP - A - 0 053 071**
**FR - A - 2 137 899**
**FR - A - 2 361 399**
**FR - A - 2 385 722**

(73) Titulaire: **RHONE-POULENC SANTE, Les Miroirs 18 Avenue d'Alsace, F-92400 Courbevoie Cedex (FR)**

(72) Inventeur: **Farge, Daniel, 30 rue des Pins Sylvestres, F-94320 Thiais (FR)**
Inventeur: **Le Roy, Pierre, 2 allée des Cerisiers, F-94320 Thiais (FR)**
Inventeur: **Moutonnier, Claude, 3 rue Auguste Rodin, F-92350 Le Plessis-Robinson (FR)**
Inventeur: **Peyronel, Jean-François, 36, Parc d'Ardenay, F-91110 Palaiseau (FR)**
Inventeur: **Plau, Bernard, 53, rue de Mesly, F-94000 Créteil (FR)**

(74) Mandataire: **Gaumont, Robert et al, RHONE-POULENC RECHERCHES Service Brevets Pharma 25, Quai Paul Doumer, F-92408 Courbevoie Cedex (FR)**

**EP 0 053 072 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouvelles oxacéphalosporines de formule générale:

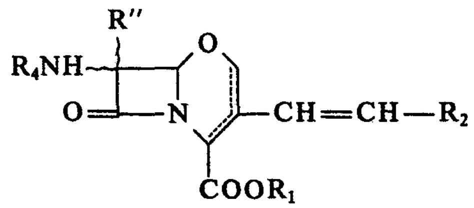

(I)

leur préparation et leur utilisation.

Dans FR-A-2 385 722 ont été décrits les produits de formule générale:

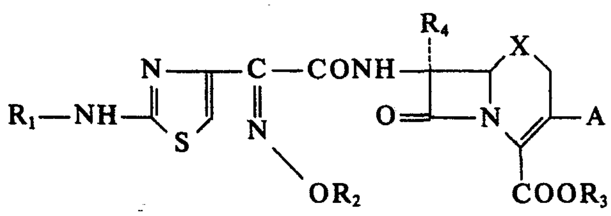

dans laquelle A représente notamment un radical alcényloxy et X représente un atome d'oxygène ou de soufre.

Dans FR-A-2 137 899 sont décrits des céphalosporines de formule générale:

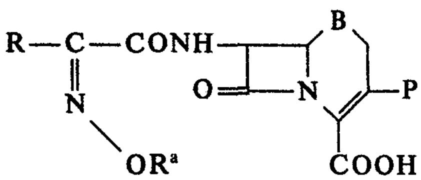

dans laquelle R peut être isothiazolyle et P représente un groupe organique notamment alcoxycarbonylvinyle.

Dans FR-A-2 361 399 ont été décrites les oxacéphalosporines de formule générale:

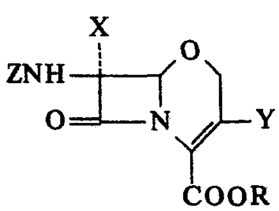

dans laquelle Z est acyle et Y est notamment un radical sulfonyloxy.

Dans la formule générale (I) les produits se présentent sous forme bicyclooctène-2- ou -3 (selon la nomenclature des Chemical Abstracts) et le substituant sur l'atome de carbone en position -3 du bicyclooctène présente la stéréoisomérie cis ou trans (désignée ci-après respectivement par Z ou E), et

a) le symbole $R_1$ représente un atome d'hydrogène ou un radical protecteur d'acide facilement éliminable (par exemple méthoxyméthyle, t. butyle, benzhydryle, benzyle, nitrobenzyle ou p.méthoxybenzyle),
le symbole $R_4$ représente un atome d'hydrogène ou un radical de formule générale:

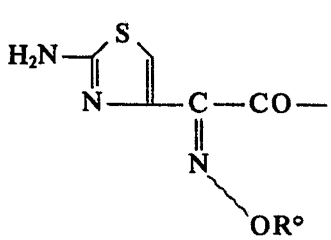

(II)

[dont la fonction amine est libre ou protégée et dans laquelle R° est un atome d'hydrogène, un

radical protecteur d'oxime, un radical alcoyle ou vinyle ou un radical carboxyalcoyle libre ou protégé représenté par la formule générale:

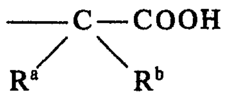 (III)

dans laquelle les radicaux $R^a$ et $R^b$, qui sont identiques ou différents, représentent des atomes d'hydrogène ou des radicaux alcoyle, ou forment ensemble un radical alcoylène contenant 2 ou 3 atomes de carbone], ou $R_4$ représente un radical $\alpha$-carboxyarylacétyle dont le groupement carboxy peut être libre ou protégé et dans lequel aryle représente phényle éventuellement substitué (par un radical p.hydroxy libre ou protégé) ou thiényle-2 ou -3, et
le symbole R″ représente un atome d'hydrogène ou un radical méthoxy en position $7\alpha$, ou bien

b) le symbole $R_1$ représente un radical protecteur d'acide facilement éliminable, le symbole $R_4$ représente un radical facilement éliminable, et le symbole R″ représente un atome d'hydrogène ou un radical méthoxy en $7\alpha$, ou un atome d'hydrogène en $7\beta$, et
le symbole $R_2$ représente un radical de formule générale:

$$-O-SO_2-R_3 \qquad (IVa)$$

ou

$$-OCO-R'_3 \qquad (IVb)$$

[dans lesquelles $R_3$ représente un radical alcoyle, trifluorométhyle, trichlorométhyle ou un radical phényle éventuellement substitué par un atome d'halogène ou par un radical alcoyle ou nitro, et $R'_3$ est défini comme $R_3$ ou représente un radical acylméthyle, acyl-2 éthyle, acyl-2 propyle, alcoyloxycarbonylméthyle, alcoyloxycarbonyl-2 éthyl ou alcoyloxycarbonyl-2 propyle] ou un atome d'halogène choisi parmi le chlore, le brome et l'iode.

Il est entendu que, sauf mention spéciale, les portions ou radicaux alcoyles ou acyles cités ci-dessus sont droits ou ramifiés et contiennent 1 à 4 atomes de carbone.

Il est également entendu que le substituant en position (-3) des produits de formule générale (I) peut se présenter sous forme E ou Z ou comme un mélange des formes E et Z.

Par radical $R_4$ facilement éliminable, on entend un radical choisi parmi:

1. benzhydryle ou trityle,

2. un radical acyle de formule générale:

$$R_5CO- \qquad (V)$$

dans laquelle $R_5$ représente:

a) un atome d'hydrogène, un radical alcoyle contenant 1 à 7 atomes de carbone, méthyle substitué par 1 à 3 atomes d'halogène, alcényle contenant 3 à 7 atomes de carbone ou cyanométhyle.
b) un radical phényle pouvant être jusqu'à 3 fois substitué (par des atomes d'halogène ou par des radicaux hydroxy, nitro, cyano, trifluorométhyle, alcoyle ou alcoyloxy) ou un radical thiényle-2 ou -3
c) un radical de formule générale:

$$R'_5\,Y\,CH_2- \qquad (VIa)$$

dans laquelle $R'_5$ représente un radical tel que défini en b) et Y représente un atome de soufre ou d'oxygène

d) un radical arylalcoyle de formule générale:

$$R''_5CH_2- \qquad (VIb)$$

dans laquelle $R''_5$ représente un radical phenyle (pouvant être jusqu'à 3 fois substitué par des radicaux hydroxy, alcoyle ou alcoyloxy) ou un radical hétérocyclyle tel que thiényle-2 ou -3, furyle-2 ou -3 ou tétrazolyle-1

3. un radical de formule générale:

$R_6OCO-$ (VII)

dans laquelle $R_6$ représente un radical alcoyle ramifié non substitué, un radical alcoyle droit ou ramifié portant un ou plusieurs substituants [tels que des atomes d'halogène ou des radicaux cyano, phényle ou phényle substitué (par un ou plusieurs atomes d'halogène ou radicaux alcoyle, alcoyloxy, nitro ou phényle)], un radical triméthylsilyl-2 éthyle, un radical vinyle ou allyle ou un radical quinolyle,

4. un radical de formule générale:

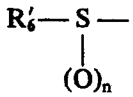 (VIII) ou $R'_6—Se—$ (IX)

dans lesquelles le reste $R'_6$ représente un radical alcoyle, phényle ou phényle substitué (par un ou hplusieurs atomes d'halogène ou radicaux nitro ou alcoyle) et n est égal à 0 ou 1,

5. un radical bis(nitro-4-benzyl)phosphoryle,

6. ou bien $R_4NH$ est remplacé par un radical dialcoylaminométhylène-amino ou par un radical de formule générale:

$Ar-CH = N-$ (X)

dans laquelle Ar représente un groupe phényle éventuellement substitué par un ou plusieurs redicaux tels que hydroxy, nitro, alcoyle ou alcoyloxy.

Comme exemples de radicaux $R_4$ pouvant être utilisés, on peut citer les radicaux suivants:

formyle, acétyle, chloracétyle, trichloracétyle, phénylacétyle,
phénoxyacétyle, benzoyle, t.butoxycarbonyle,
chloro-2 diméthyl-1,1 éthoxycarbonyle
trichloro-2,2,2 éthoxycarbonyle,
trichloro-2,2,2 diméthyl-1,1 éthoxycarbonyle,
cyano-2 diméthyl-1,1 éthoxycarbonyle,
triméthylsilyl-2 éthoxycarbonyle,
benzyloxycarbonyle,
p.méthoxybenzyloxycarbonyle,
diméthoxy-3,5 benzyloxycarbonyle,
p.nitrobenzyloxycarbonyle,
diphénylméthoxycarbonyle,
(biphénylyl-4)-2 isopropyloxycarbonyle,
vinyloxycarbonyle,
allyloxycarbonyle,
quinolyl-8 oxycarbonyle,
o.nitrophénylthio,
p.nitrophénylthio,
bis(nitro-4 benzyl) phosphoryle.

Comme exemples de radicaux méthylène amino définis précédemment en 6), on peut citer:

diméthylaminométhylèneamino,
diméthoxy-3,4 benzylidèneamino,
nitro-4 benzylidèneamino,
di t.butyl-3,5 hydroxy-4 benzylidèneamino.

Par ailleurs, il est entendu que le groupement OR° du radical de formule générale (II) peut se trouver dans l'une des positions syn ou anti et que ces isomères et leurs mélanges entrent dans le cadre de la présente invention.

La forme syn peut être représentée par la formule:

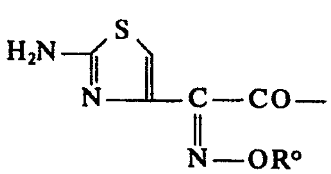 (IIa)

La forme anti peut être représentée par la formule:

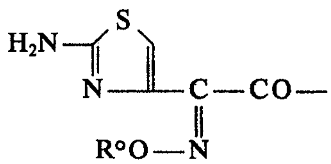

(IIb)

Lorsque $R_4$ représente un radical de formule générale (II) parmi les significations préférées du symbole R° on peut citer notamment: méthyle, éthyle, propyle, isopropyle, butyle, isobutyle, sec.butyle, vinyle, carboxyméthyle libre ou protégé, carboxy-2 propyle libre ou protégé, ou radical protecteur [choisi par exemple parmi trityle, tétrahydropyrannyle, méthoxy-2 propyle-2, alcoyloxycarbonyle, (tel que t.butoxycarbonyle) ou aryloxycarbonyle (tel que benzyloxycarbonyle)].

Lorsque R° contient un radical carboxy protégé, le radical protecteur peut être choisi par exemple parmi méthoxyméthyle, t.butyle, benzhydryle, benzyle, nitrobenzyle ou p.méthoxybenzyle.

Lorsque $R_4$ est un radical de formule générale (II) la fonction amine de ce radical peut être protégée par un radical tel que t.butoxycarbonyle, trichloro-2,2,2 éthoxycarbonyle, trichloracétyle, trityle, benzyle, dibenzyle benzyloxycarbonyle, p.nitrobenzyloxycarbonyle, p.méthoxybenzyloxycarbonyle, chloracétyle, formyle ou trifluoracétyle.

Lorsque $R_4$ représente un radical $\alpha$-carboxyarylacétyle dont le groupement carboxy est protégé, ce dernier peut être protégé par un radical protecteur d'acide, par exemple tel que cité ci-dessus pour R°.

Lorsque $R_4$ représente un radical $\alpha$-carboxy p.hydroxyphénylacéthyle dont le groupement hydroxy est protégé, ce dernier peut être protégé par exemple par un radical tel que cité ci-dessus comme protecteur d'oxime pour R° .

Selon l'invention les produits de formule générale (I) dans laquelle $R_1$, $R_2$, $R_4$ et R″ sont définis comme ci-dessus, à l'exception pour $R_4$ de représenter un atome d'hydrogène, peuvent être préparés par action d'un dérivé activé des acides $R_3SO_3H$ et $R'_3COOH$ du type:

$$\left.\begin{array}{ll} (R_3SO_2)_2O & (a) \\ R_3SO_2Hal & (b) \\ (R'_3CO)_2O & (c) \\ R'_3COHal & (d) \end{array}\right\} \qquad (XI)$$

($R_3$ et $R'_3$ étant définis comme précédemment et Hal représentant un atome d'halogène), ou d'un agent d'halogénation, sur un produit de formule générale:

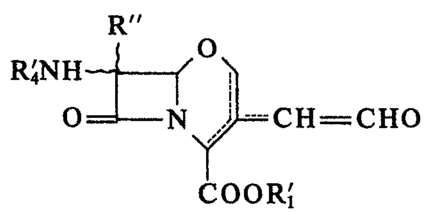

(XII)

(ou un mélange de ses isomères) dans laquelle, $R'_1$ étant défini comme $R_1$ précédemment en b), le produit se présente sous forme bicyclooctène-2 ou -3 ou oxoéthylidène-3 bicyclooctane et
soit $R'_4$ est défini comme $R_4$ dans la formule générale (I) en a), étant entendu que lorsqu'il représente un radical de formule générale (II), l'amine de ce dernier est protégée,
et R″ est un atome d'hydrogène ou un radical méthoxy en position $7\alpha$, soit $R'_4$ est défini comme $R_4$ dans la formule générale (I) en b) et R″ est un atome d'hydrogène ou un radical méthoxy en $7\alpha$ ou un atome d'hydrogène en $7\beta$, suivie éventuellement de l'élimination des radicaux protecteurs.

Lorsque l'on désire mettre en oeuvre un produit de formule générale (XII) dans laquelle $R'_4$ est un radical de formule générale (II) dont le reste R° représente un atome d'hydrogène, il est nécessaire de protéger préalablement l'oxime.

Lorsque l'on désire mettre en oeuvre un aldéhydre de formule générale (XII) dans laquelle $R'_4$ contient un groupement carboxy, ce radical peut être libre ou protégé si l'on fait agir un dérivé activé des acides $R_3SO_3H$ ou $R'_3COOH$; par contre il est nécessaire de le protéger préalablement si l'on fait agir un agent d'halogénation.

Lorsque l'on désire mettre en oeuvre un aldéhyde dans lequel le radical $R'_4$ représente $\alpha$-carboxy (p.hydroxyphényl) acétyle, il est nécessaire de protéger le adical hydroxy.

La protection des radicaux s'effectue dans les conditions décrites précédemment.

On opère généralement en présence d'une base teriaire telle que définie par la formule générale:

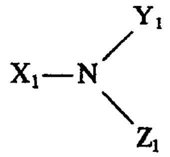

(XIII)

[dans laquelle $X_1$, $Y_1$ et $Z_1$ représentent des radicaux alcoyle ou phényle, ou éventuellement deux d'entre eux forment un cyclo avec l'atome d'azote auquel ils sont rattachés], par exemple la triéthylamine ou la NN-diméthylaniline, dans un solvant organique chloré (par exemple dichlorméthane), dans un ester (acétate d'éthyle), dans un éther (par exemple dioxanne, tétrahydrofuranne), dans un amide (par exemple diméthylacétamide, diméthylformamide), dans l'acétonitrile ou la N-méthylpyrrolidone ou dans un mélange de tels solvants, ou directement dans un solvant basique comme la pyridine ou bien, lorsque $R_2$ est autre qu'un atome d'halogène, on peut opérer en milieu hydroorganique en présence d'un agent alcalin de condensation (par exemple bicarbonate alcalin, soude ou potasse), à une température comprise entre −78° C et la température de reflux du mélange réactionnel.

Eventuellement on opère sous azote.

Il n'est pas absolument nécessaire d'avoir purifié l'intermédiaire de formule générale (XII) pour mettre en oeuvre cette réction.

Lorsque l'on veut préparer un produit de formule générale (I) dans laquelle $R_2$ est un atome d'halogène, l'agent d'halogénation peut être choisi parmi les dérivés halogénés du phosphore, notamment:

— les composés d'addition d'halogène et de triarylphosphite, ou bien
— le trichlorure de phosphore, l'oxychlorure de phosphore, le pentachlorure de phosphore, le dichlorotriphénylphosphorane ou le catéchyltrichlorophosphorane lorsque $R_2$ est un atome de chlore, ou
— le tribromure de phosphore, l'oxybromure de phosphore, le pentabromure de phosphore ou le catéchylbromophosphorane lorsque $R_2$ représente un atome de brome.

Le catéchyltrichloro (ou tribromo) phosphorane, qui peut être préparé in situ, peut être obtenu selon la méthode décrite par H. GROSS et U. KARSCH, J. Prakt. Chem. 29, 315 (1965).

Les composés d'addition d'halogène et de triarylphosphite, qui peuvent être préparés in situ, sont décrits par H. N. RYDON et B. L. TONGE, J. Chem. Soc., 3043 (1956), par J. MICHALSKI et coll., J. Org. Chem., 45, 3122 (1980) ou dans BE-A-881 424 et peuvent être préparés selon les méthodes mentionnées dans ces documents.

La préparation des dérivés halogénés de formule générale (I) s'effectue en milieu anhydre.

Lorsque l'on veut préparer un produit de formule générale (I), dans laquelle $R_2$ représente un atome de chlore ou de brome, selon les conditions opératoires on peut isoler l'intermédiaire dihalogéné de formule générale:

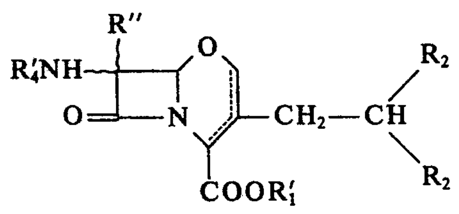

(XIV)

[dans laquelle R″, $R'_4$, $R'_1$, et $R_2$ étant définis comme ci-dessus, le produit présente la même isomérie que le produit de formule générale (I)] qui est ensuite déhydrohalogéné.

Lorsque l'on veut isoler l'intermédiaire dihalogéné, on opère par action d'un agent d'halogénation, dans un solvant organique tel qu'un solvant chloré (par exemple dichlorométhane, chloroforme, tétrachlorure de carbone, dichloro-1,2 éthane), un éther (par exemple éther éthylique, oxyde de propylène, tétrahydrofuranne, dioxanne), un amide (par exemple diméthylacétamide, diméthylpropionamide, diméthylformamide, N-acétylmorpholine, N-acétylpipéridine, N-méthylpyrrolidone) ou un mélange de tels solvants, à une température un peu moins élevée que pour préparer le dérivé halogénovinyle correspondant, c'est-à-dire comprise entre −78 et 30° C.

Il est également possible d'opérer en présence d'une base telle que la pyridine dans un solvant cité ci-dessus, à une température comprise entre −78 et 0° C.

La déhydrohalogénation s'effectue en présence d'une base tertiaire telle que définie précédemment, d'une amine aromatique (par exemple pyridine, picoline, quinoléine) ou d'une base minérale (telle que la soude, la potasse, un carbonate ou un bicarbonate alcalin ou alcaline-terreux), en milieu

organique ou hydroorganique dans les solvants cités précédemment, à une température comprise entre —20° C et la température de reflux du mélange réactionnel.

Il n'est pas absolument nécessaire d'avoir purifié l'intermédiaire dihalogéné pour procéder à sa déhydrohalogénation.

L'élimination des différents radicaux protecteurs peut s'effectuer simultanément ou successivement.

A titre d'exemple, 1/L'élimination des groupements protecteurs d'amines s'effectue

— lorsqu'il s'agit d'un radical t.butoxycarbonyle, trityle, p.méthoxybenzyloxycarbonyle ou formyle: par traitement en milieu acide. On utilise par exemple l'acide trifluoracétique en opérant à une température comprise entre 0 et 20° C, ou bien on utilise l'acide formique, phosphorique ou polyphosphorique purs ou en présence d'eau à une température comprise entre 20 et 60° C, ou encore l'acide p.toluènesulfonique ou méthanesulfonique dans l'acétone ou l'acétonitrile, à une température comprise entre 20° C et la température de reflux du mélange réactionnel. Dans ces conditions lorsque $R_4$ est un radical de formule générale (II) le produit de formule générale (I) peut être obtenu sous forme de trifluoracétate, de solvate avec l'acide formique, de phosphate, de méthanesulfonate ou de p.toluènesulfonate, dont on peut libérer la fonction amine par toute méthode connue en soi pour obtenir une amine à partir de l'un de ses sels sans toucher au reste de la molécule. On opère notamment par mise en contact avec une résine échangeuse d'ions ou par action d'une base organique;
— lorsqu'il s'agit d'un radical trichloro-2,2,2 éthoxycarbonyle ou p.nitrobenzyloxycarbonyle : par réduction (notamment traitement par le zinc dans l'acide acétique);
— lorsqu'il s'agit d'un radical chloracétyle ou trichloracétyle: par application de la méthode décrite dans FR-A-2 243 199;
— lorsqu'il s'agit d'un radical benzyle, dibenzyle ou benzyloxycarbonyle: par hydrogénation catalytique;
— lorsqu'il s'agit d'un radical trifluoracétyle: par traitement en milieu basique.

2/ L'élimination des groupements protecteurs du radical carboxy s'effectue:
— lorsqu'il s'agit d'un groupement t.butyle, p.méthoxybenzyle ou benzhydryle: par traitement en milieu acide, dans les conditions décrites ci-dessus pour l'élimination du radical trityle protecteur d'amino. Dans le cas du radical benzhydryle, on peut opérer en présence d'anisole;
— lorsqu'il s'agit d'un groupement méthoxyméthyle: par traitement en milieu acide dilué;
— lorsqu'il s'agit d'un groupement nitrobenzyle: par réduction (notamment traitement par le zinc dans l'acide acétique ou hydrogénolyse).

3/ L'élimination des groupements protecteurs de l'oxime ou du radical hydroxy s'effectue:

— lorsqu'il s'agit de groupement trityle ou tétrahydropyrannyle: par acidolyse, par exemple par l'acide trifluoracétique, l'acide formique aqueux ou non, ou l'acide p.toluènesulfonique;
— lorsqu'il s'agit du groupement méthoxy-2 propyle-2: selon la méthode décrite dans BE-A-875 379;
— lorsqu'il s'agit des groupements alcoyloxycarbonyle ou aryloxycarbonyle: selon les méthodes décrites dans le brevet belge 871 213.

Les produits de formule générale (XII) peuvent être obtenus par hydrolyse de l'énamine (ou du mélange d'énamines isomères) de formule générale:

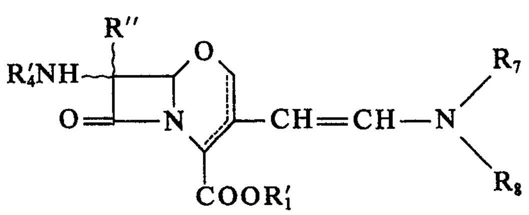

(XV)

dans laquelle R'', $R'_1$ et $R'_4$ étant définis comme précédemment pour la formule générale (XII) (étant entendu que, le cas échéant, les radicaux amino et/ou carboxy contenus par $R'_4$ sont protégés), le produit se présente sous forme bicyclooctène-2 ou -3, le substituant sur l'atome de carbone en position -3 du bicyclooctène présente la stéréoisomérie E ou Z, et
$R_7$ et $R_8$ qui sont identiques ou différents représentent des radicaux alcoyle (éventuellement substitués par un radical alcoyloxy ou dialcoylamino) ou phényle, ou forment ensemble avec l'atome d'azote auquel ils sont rattachés un hétérocycle saturé à 5 ou 6 chaînons, contenant éventuellement un autre hétéroatome choisi parmi l'azote, l'oxygène ou le soufre et éventuellement substitué par un radical alcoyle.

On opère généralement dans un acide organique (par exemple acide formique, acide acétique) ou minéral (par exemple acide chlorhydrique, acide sulfurique) en présence ou non d'un solvant, en milieu aqueux ou organique, à une température comprise entre —20°C et la température de reflux du

mélange réactionnel. Lorsque l'on opère en milieu organique, l'hydrolyse est réalisée par addition d'eau au mélange réactionnel, puis on traite éventuellement par une base minérale (par exemple bicarbonate alcalin) ou organique (par exemple amine tertiaire ou pyridine).

Lorsque l'on opère en présence d'un solvant, il n'est pas nécessaire que le solvant soit miscible à la phase aqueuse acide. Le contact est alors réalisé par agitation vive.

Parmi les solvants utilisables, peuvent être cités les solvants chlorés, l'acétate d'éthyle, le tétrahydrofuranne, l'acétonitrile, le diméthylformamide, les alcools. Il n'est pas absolument nécessaire d'avoir purifié l'intermédiaire de formule générale (XV) pour mettre en oeuvre cette réaction.

Lorsque l'on veut obtenir un aldéhyde de formule générale (XII) dans laquelle $R'_4$ contient une fonction acide libre, il est nécessaire d'opérer à partir d'une énamine dans laquelle $R'_1$ et le groupement protecteur de la fonction acide de $R'_4$ sont différents et éliminables sélectivement.

L'élimination du radical protecteur s'effectue dans les conditions décrites précédemment.

Les produits de formule générale (XV) peuvent être obtenus par action d'un produit de formule générale:

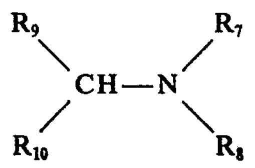

(XVI)

éventuellement préparé in situ [pour lequel $R_7$ et $R_8$ sont définis comme précédemment et $R_9$ et $R_{10}$ qui sont identiques ou différents soit représentent des groupements de formule générale:

$$-X_2R_{11} \qquad \text{(XVIIa)}$$

dans laquelle $X_2$ est un atome d'oxygène et $R_{11}$ représente un radical alcoyle ou phényle, soit représentent l'un un radical de formule générale (XVIIa) (dans lequel $X_2$ représente un atome d'oxygène ou de soufre et $R_{11}$ est alcoyle ou phényle), et l'autre un radical amino de formule générale:

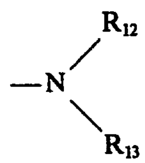

(XVIIb)

dans laquelle $R_{12}$ et $R_{13}$ sont définis comme $R_7$ et $R_8$, soit encore $R_9$ et $R_{10}$ représentent chacun un radical de formule générale (XVIIb)] sur un dérivé d'oxacéphalosporine de formule générale:

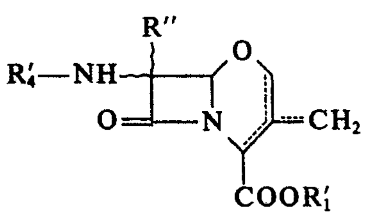

(XVIII)

dans laquelle, R″, $R'_1$ et $R'_4$ étant définis comme précédemment dans la formule générale (XV), le produit se présente sous forme méthyl-3 bicyclooctène-2 ou -3 ou méthylène-3 bicyclooctane.

Lorsque l'on choisit un produit de formule générale (XVI) dans laquelle le radical (XVIIb) est différent de $-NR_7R_8$, il est préférable de choisir un tel produit de manière que l'amine $HNR_{12}R_{13}$ soit plus volatile que $HNR_7R_8$.

On opère généralement dans un solvant organique tel qu'un amide (par exemple le diméthylformamide, le diméthylacétamide ou l'hexaméthylphosphorotriamide), un nitrile (par exemple l'acétonitrile), un ester (par exemple l'acétate d'éthyle), un éther (par exemple le dioxanne), un solvant chloré (par exemple le dichloro-1,2 éthane) ou encore dans un mélange de tels solvants à une température comprise entre 20° C et la température de reflux du mélange réactionnel.

Il est entendu que lorsque $R'_4$ représente un radical de formule générale (II) dans laquelle R° représente un atome d'hydrogène, il est préférable que l'oxime soit protégée dans les conditions décrites précédemment.

Il est également entendu que lorsque $R'_1$ contient un substituant hydroxy, il est préférable de protéger ce dernier.

La protection, et l'élimination des radicaux protecteurs, s'effectuent dans les conditions décrites précédemment.

les produits de formule générale (XVI) peuvent être préparés selon les méthodes décrites par H. BREDERECK et coll., Chem. Ber. 101 41 (1968), Chem. Ber. 101, 3058 (1968) et Chem. Ber. 106, 3725

(1973).Les dérivés de l'oxacéphalosporine de formule générale (XVIII) dans laquelle R'$_4$ représente un radical de formule générale (II) ou un radical $\alpha$-carboxyarylacétyle dont les fonctions amine et/ou acide sont protégées peuvent être préparés à partir des produits de formule générale:

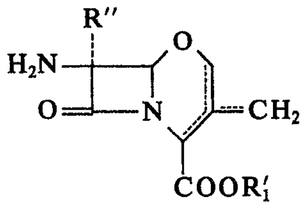

(XIX)

dans laquelle R'$_1$ est défini comme précédemment et R'' représente un atome d'hydrogène ou un radical méthoxy en position 7$\alpha$, en opérant par action d'un acide de formule générale:

R'$_4$—OH (XX)

dans laquelle R'$_4$ est défini comme ci-dessus, ou d'un dérivé réactif de cet acide, en opérant dans les conditions décrites ci-après pour la préparation des produits de formule générale (I) au moyen d'un acide de formule générale (XXI) ou d'un de ses dérivés réactifs.

Les conditions de déblocage des substituants sont telles que décrites précédemment pour la préparation des oxacéphalosporines de formule générale (I).

Les oxacéphalosporines de formules générales (XVIII) et (XIX) peuvent être préparées selon ou par analogie avec les méthodes décrites dans la littérature, par exemple:

— lorsque R'' représente un atome d'hydrogène: selon les méthodes décrites par C. L. BRANCH et coll., J. C. S. Perkin I, 2268 (1979) dans DE-A-2 806 457, dans US-A-4 108 992, dans DE-A-2 355 209, puis éventuellement mise en place du radical R'$_4$ (lorsque l'on veut obtenir un produit de formule générale (XVIII), par analogie avec les méthodes employées en chimie des céphalosporines et par exemple:
— lorsque R'$_4$ est un radical formyle: selon J. C. SHEEHAN et coll., J. Amer. Chem. Soc. 80 1154 (1958)
— lorsque R'$_4$ est acétyle, chloracétyle, trichloracétyle, phénylacétyle, phénoxyacétyle ou benzoyle: selon E. H. FLYNN, Cephalosporins and Pinicillins, Ac. Press (1972),
— lorsque R'$_4$ est un radical t.butoxycarbonyle: selon L. MORODER et coll., Hoppe Seyler's Z. Physiol. Chem. 357 1651 (1976),
— lorsque R'$_4$ est trichloro-2,2,2 diméthyl-1,1 éthoxycarbonyle: selon J. UGI et coll., Angew. Chem. Int. Ed. Engl. 17(5) 361 (1978),
— lorsque R'$_4$ est trichloro-2,2,2 éthoxycarbonyle, chloro-2 diméthyl-1,1 éthoxycarbonyle, cyano-2, diméthyl-1,1 éthoxycarbonyle, triméthylsilyl-2 éthoxycarbonyle, benzyloxycarbonyle, p.méthoxybenzyloxycarbonyle, diméthoxy-3,5 benzyloxycarbonyle, p.nitrobenzyloxycarbonyle, vinyloxycarbonyle: par action d'un chloroformiate en milieu hydroorganique en présence d'un bicarbonate alcalin, ou selon BE-A-788 885,
— lorsque R'$_4$ est diphénylméthoxycarbonyle: par action de l'azidoformiate correspondant en milieu hydroorganique, en présence d'un bicarbonate alcalin,
— lorsque R'$_4$ est (biphényl-4)-2 isopropyloxycarbonyle: par analogie avec la méthode décrite dans Helv. Chim. Acta, 51, 924 (1968),
— lorsque R'$_4$ est quinolyl-8 oxycarbonyle ou allyloxycarbonyle: par action du carbonate correspondant en milieu hydroorganique basique,
— lorsque R'$_4$ est o.nitrophénylthio ou p.nitrophénylthio: par analogie avec la méthode décrite par T. KOBAYASHI et coll., Chem. Pharm. Bull. 27(11), 2718 (1979),
— lorsque R'$_4$NH est remplacé par diméthylaminométhylèneamino: par analogie avec la méthode décrite par J. F. FITT, J. Org. Chem. 42(15), 2639 (1977),
— lorsque R'$_4$NH est remplacé par nitro-4 benzylidèneamino ou diméthoxy-3,4 benzylidèneamine: selon la méthode décrite par R. A. FIRESTONE, Tetrahedron Lett., 375 (1972),
— lorsque R'$_4$NH est remplacé par di t.butyl-3,5 hydroxy-4 benzylidèneamino: selon la méthode décrite par H. YANAGISAWA et coll., Tetrahedron Lett., 2705 (1975),
— lorsque R'$_4$ est bis(nitro-4 benzyl)phosphoryle: selon la méthode décrite dans la demande de brevet japonais 77 125 185,
— lorsque R'' représente un radical méthoxy: par analogie avec les méthodes décrites ci après pour la préparation des produits de formule générale (XXVIII) ou (XXVII) ou selon la demande de brevet allemand 2 806 457 puis, lorsque l'on veut obtenir un produit de formule générale (XVIII), mise en place du radical R'$_4$ par analogie avec les méthodes citées ci-dessus.

Les produits de formule générale (XX) peuvent être préparés selon la méthode décrite dans le brevet belge 850 662, ou par application de la méthode décrite dans le brevet belge 877 884, lorsque R'$_4$ est un radical de formule générale (II) dans laquelle R° est hydrogène ou alcoyle.

Les produits de formule générale (XX) peuvent être préparés selon la méthode décrite dans le brevet belge 869 079 lorsque R'$_4$ est un radical de formule générale (II) dans laquelle R° est vinyle.

Les produits de formule générale (XX) peuvent être préparés selon les méthodes décrites dans les brevets belges 864 810, 865 298, 876 541 et 876 542 lorsque R'$_4$ est un radical de formule générale (II) dans laquelle R° est un substituant de formule générale (III).

Les produits de formule générale (XX), lorsque R'$_4$ est un radical $\alpha$-carboxyarylacétyle, peuvent être préparés selon les méthodes suivantes:

— lorsque le groupement aryle représente un groupement p.hydroxyphényle: selon la méthode décrite dans JP-A-79 106 447 ou dans BE-A-852 912.
— lorsque le groupement aryle représente un groupement thiényle-2: selon D. INVANOV et N. MAREKOV: Compt. Rend. Acad. Bulgare Sci., 8(11), 29 (1955).
— lorsque le groupement aryle représente un groupement thiényle-3: selon GB-A-1 125 557.

Selon l'invention, les produits de formule générale (I) dans laquelle R$_1$, R$_2$ et R'' sont définis comme précédemment en a) et R$_4$ est un atome d'hydrogène peuvent être obtenus par élimination du radical protecteur R$_4$ d'un produit de formule générale (I) dans laquelle R$_1$, R$_2$ et R$_4$ sont définis comme précédemment en b) et R'' est situé en position 7$\alpha$ (ou éventuellement par élimination simultanée des radicaux protecteurs R$_1$ et R$_4$ lorsque l'on veut obtenir un produit de formule générale (I) pour lequel R$_1$ et R$_4$ sont des atomes d'hydrogène).

L'élimination du radical protecteur R$_4$ s'effectue par toute méthode connue pour libérer une fonction amine sans toucher au reste de la molécule.

A titre d'exemple, on peut citer les méthodes suivantes:

— lorsque R$_4$ représente trityle, benzhydryle, trichloracétyle, chloracétyle, t.butoxycarbonyle, trichloréthoxycarbonyle, benzyloxycarbonyle, p.méthoxybenzyloxycarbonyle et p.nitrobenzyloxycarbonyle: selon les méthodes citées ci-dessus pour la libération du radical amino du produit de formule générale (I);
— lorsque R$_4$ représente formyle, chloro-2 diméthyl-1,1 éthoxycarbonyle, cyano-2 diméthyl-1,1 éthoxycarbonyle, diméthoxy-3,5 benzyloxycarbonyle, diphénylméthoxycarbonyle, (biphénylyl-4)-2 isopropyloxycarbonyle, vinyloxycarbonyle, allyloxycarbonyle, quinolyl-8 oxycarbonyle, o.nitrophénylthio, p.nitrophénylthio, et lorsque R$_4$ NH— est remplacé par diméthylèneamino, diméthoxy-3,4 benzylidèneamino ou nitro-4 benzylidèneamino: par acidolyse,
— lorsque R$_4$ représente di t.butyl-3,5 hydroxy-4 benzylidèneamino: par traitement par le réactif T de Girard par analogie avec la méthode décrite dans BE-A-863 998,
— lorsque R$_4$ représente trichloro-2,2,2 éthyle ou trichloro-2,2,2 diméthyl-1,1 éthoxycarbonyle: par traitement par le zinc dans l'acide acétique,
— lorsque R$_4$ représente acétyle, benzoyle, phénylacétyle ou phénoxyacétyle: selon la méthode décrite dans BE-A-758 800 ou selon la méthode décrite par YOXHIOKA, Tet. Letters 351 (1980),
— lorsque R$_4$ représente triméthylsilyléthoxycarbonyle: selon la méthode décrite par H. GERLACH, Helv. Chim. Acta 60(8), 3039 (1977),
— lorsque R$_4$ représente p.nitrobenzyloxycarbonyle et benzyle: par hydrogénolyse en présence de palladium,
— lorsque R$_4$ représente un radical bis(nitro-4 benzyl)phosphoryle: par application de la méthode décrite dans JP-A-77 125 185.

Lorsque R'' est un groupe méthoxy on pourra, de préference aux méthodes précédentes, utiliser les méthodes suivantes:

1. Dans le cas où R$_4$ représente benzhydryle ou trityle: hydrogénolyse en présence de palladium.
2. Dans le cas où R$_4$ représente des radicaux de formules générales (VIII) ou (IX): par les méthodes décrites par E. M. GORDON, J. Am. Chem. Soc. 102(5), 1690 (1980); T. KOBAYASHI, Bull. Chem. Soc. Japan 52(11), 3366 (1979)) et T. KOBAYASHI, chem. Pharm. Bull. 27, 2718 (1979).
3. Dans le cas où R$_4$ représente un radical de formule générale (X): par acidolyse ou par traitement par le réactif T de Girard, par analogie avec la méthode décrite dans le brevet belge 863 998.

Selon l'invention, les produits de formule générale (I) dans laquelle R$_1$, R$_2$, R$_4$ et R'' sont définis comme précédemment en a) (à l'exception pour R$_4$ de représenter un atome d'hydrogène) peuvent également être obtenus par action d'un acide de formule générale:

$$R_4-OH \qquad (XXI)$$

[dont la fonction amine est préalablement protégée lorsque R$_4$ est un radical de formule générale (II)], ou par action d'un de ses dérivés réactifs, sur une amino-7 oxacéphalosporine de formule générale (I) dans laquelle R$_4$ est un atome d'hydrogène et R$_1$, R$_2$ et R'' sont définis comme en a), puis éventuellement élimination des radicaux protecteurs.

a) Lorsque l'on utilise le produit de formule générale (XXI) sous forme acide, on effectue généralement la condensation de ce produit (dont le cas échéant les fonctions amine et/ou oxime ont été préalablement protégées) sur l'amino-7 oxacéphalosporine de formule générale (I) dans laquelle $R_1$ représente un radical protecteur facilement éliminable en opérant dans un solvant organique tel que le diméthylformamide, l'acétonitrile, le tétrahydrofuranne, le dichlorométhane ou le chloroforme, en présence d'un agent de condensation tel qu'un carbodiimide (par exemple le dicyclohexylcarbodiimide), le NN'-carbonyl-diimidazole ou l'éthoxy-2 éthoxycarbonyl-1 dihydro-1,2 quinoléine, à une température comprise entre −20 et 40°C puis on élimine les groupements protecteurs présents dans la molécule. Eventuellement, on opère en présence d'une quantité catalytique de N,N-diméthylamino-4 pyridine.

b) Lorsque l'on utilise un dérivé réactif de l'acide de formule générale (XXI), il est possible de mettre en oeuvre l'anhydride, un anhydride mixte ou un ester réactif de formule générale:

$R_4-OZ$ (XXII)

dans laquelle $R_4$ est défini comme ci-dessus et Z représente un radical succinimido, benzotriazolyle-1, nitro-4 phényle, dinitro-2,4 phényle, pentachlorphényle ou phtalimido. Lorsque $R_4$ est un radical de formule générale (II), la fonction amine de tels dérivés est préalablement protégée (par exemple comme décrit précédemment). Les conditions de protection des différents substituants sont telles que décrites précédemment.

Il est également possible de mettre en oeuvre un dérivé réctif tel qu'un halogénure d'acide. Dans ce derni er cas, lorsque $R_4$ est un radical de formule générale (II) on peut, par exemple , faire réagir le chlorhydrate du chlorure d'acide sur l'amino-7 oxacéphalosporine de formule générale (I). Lorsque $R_4$ est un radical $\alpha$-carboxy (p.hydroyphényl) acétyle, le groupement hydroxy peut être libre ou protégé.

Lorsque l'on met en oeuvre l'anhydride, un anhydride mixte ou un halogénure d'acide (qui peuvent être préparés in situ), on effectue la condensation dans un solvant organique inerte tel qu'un éther (par exemple tétrahydrofuranne ou dioxanne), un solvant chloré (par exemple chloroforme ou chlorure de méthylène), un amide (par exemple diméthylformamide ou diméthylacétamide) ou une cétone (par exemple acétone), ou dans des mélanges de tels solvants en présence d'un accepteur d'acide tel qu'un époxyde (par exemple l'oxyde de propylène) ou tel qu'une base organique azotée comme la pyridine, la diméthylaminopyridine, la N-méthylmorpholine ou une trialcoylamine (par exemple triéthylamine), ou dans un milieu hydroorganique en présence d'un agent alcalin de condensation tel que le bicarbonate de sodium, et l'on opère à une température comprise entre −40 et +40°C, puis on remplace éventuellement les groupements protecteurs par des atomes d'hydrogène.

Lorsque l'on met en oeuvre un ester réactif de formule générale (XXII), on opère généralement en présence d'une trialcoylamine (par exemple thiéthylamine) dans un solvant organique tel que le diméthylformamide à une température comprise entre 0 et 40°C, puis on remplace les groupements protecteurs par des atomes d'hydrogène.

Le cas échéant , l'élimination des radicaux protecteurs peut être efffectuée dans les conditions décrites précédemment.

Les nouveaux produits de formule générale (I) sont utiles comme intermédiaires pour la préparation d'oxacéphalosporines de formule générale:

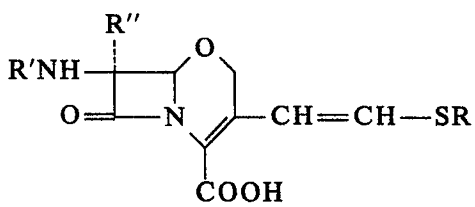

(XXIII)

dans laquelle le symbole R est choisi parmi les significations:

1. pyridyle-2, -3 ou -4 éventuellement N-oxydés
2. pyrmidinyle-2,
3. méthyl-6 oxyde-1 pyridazinyle-3,
4. dioxo-5,6 tètrahydro-1,4,5,6 triazine-1,2,4 yle-3, substitué en position -4 par
   a) un radical alcoyle contenant 1 ou 2 atomes de carbone éventuellement substitué par un radical alcoyloxy, alcoylthio, formyle,
   b) un radical allyle, dihydroxy-2,3 propyle, dihydroxy-1,3 propyle-2 ou formyl-2 hydroxy-2 éthyle,
   c) un radical alcoyle contenant 2 ou 3 atomes de carbone substitué par hydroxy, carbamoyloxy, acyloxy ou acylamino (dont les portions acyles sont non substituées ou substituées par amino), alcoylsulfonylamino, uréido, alcoyluréido ou dialcoyluréido,

5. dioxo-5,6 tétrahydro-1,4,5,6 triazine-1,2,4 yle-3 substitué en position -1 ou dioxo-5,6 tétrahydro-1,2,5,6 triazine-1,2,4 yle-3 substitué en position -2 par un radical alcoyle contenant 1 ou 2 atomes de carbone éventuellement substitué par un radical formyle,
6. alcoyl-2 dihydro-2,5 oxo-5 triazine-1,2,4 yle-3 éventuellement substitué en position -6 par un radical alcoyle, alcoyloxy dont les portions et radicaux alcoyles contiennent 1 ou 2 atomes de carbone
7. amine-1 dihydro-1,2 oxo-2 pyrimidinyle-4,
8. thiadiazol-1,3,4 yle-5 substitué par alcoyle, dialcoylaminoalcoyle ou acylaminoalcoyle,
9. tétazolyle-5 substitué en position-1 par,
   a) un radical alcoyle contenant 1 ou 2 atomes de carbone éventuellement substitué par un radical formyle,
   b) un radical alcoyle contenant 2 ou 3 atomes de carbone substitué par hydroxy, acylamino, ou dialcoylamino, ou
   c) par un radical dihydroxy-2,3 propyle ou dihydroxy-1,3 propyle-2 10)a) alcoyl-1 triazol-1,2,4 yle-5 éventuellement substitué en position -3 par un radical alcoyloxycarbonyle dont les radicaux alcoyle et alcoyloxy contiennent 1 ou 2 atomes de carbone,

b) alcoyl-1 triazol-1,3,4 yle-5,

le symbole R' représente un radical formule générale (II) [dans laquelle R° est un atome d'hydrogène, un radical alcoyle, vinyle ou carboxyalcoyle tel que défini par la formule générale (III)] ou représente un radical $\alpha$-carboxyarylacéthyle dans lequel aryle représente phényle (éventuellement substitué par un radical p.hydroxy) ou thiényle-2 ou -3 et,
le symbole R'' représente un atome d'hydrogène ou un radical méthoxy en $7\alpha$,
et dans laquelle le substituant en position -3 se présente sous les formes E ou Z.

Les produits de formule générale (XXIII) dont la préparation est décrite plus en détail dans la demande européenne parallèle publiée sous le n° EP-A-53 071 peuvent être obtenus à partir des produits de formule générale (I) en opérant comme suit:

1° — Les oxacéphalosporines de formule générale (XXIII) peuvent être préparées par action d'un thiol de formule générale:

$$R{-}SH \qquad \text{(XXIV)}$$

(ou d'un de ses sels alcalins ou alcalinoterreux) dans laquelle R est défini comme ci-dessus [[R étant protégé à l'état d'acétal, sous forme d'un radical de formule générale:

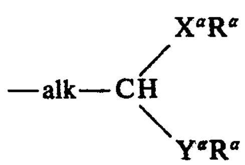 (XXVa) ou 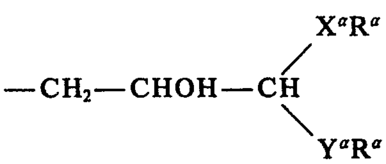 (XXVb)

[dans lesquelles alk est un radical alcoylène contenant 1 à 4 atomes de carbone, $X^{\alpha}$ et $Y^{\alpha}$ sont identiques et représentant des atomes d'oxygène ou de soufre, et $R^{\alpha}$ représente un radical alcoyle ou bien $X^{\alpha}$ et $Y^{\alpha}$ sont identiques ou différents et représentent des atomes d'oxygène ou de soufre, et les radicaux $R^{\alpha}$ forment ensemble un radical alcoylène contenant 2 ou 3 atomes de carbone], lorsque l'on veut obtenir une oxacéphalosporine de formule générale (XXIII) dans laquelle R contient un radical formyle]] sur un dérivé d'oxacéphalosporine (ou le cas échéant sur un mélange des isomères de ce dérivé) de formule générale (I) dans laquelle $R_1$, $R_2$, $R_4$ et R'' sont définis comme précédemment en a), à l'exception pour $R_4$ de représenter un atome d'hydrogène, suivie le cas échéant de l'élimination des radicaux protecteurs.

2° — Les oxacéphalosporines de formule générale (XXIII) peuvent également être obtenues de la manière suivante:

On fait agir un thiol de formule générale (XXIV) dont le radical R est éventuellement protégé, ou un de ses sels alcalins ou alcalinoterreux, sur un dérivé d'oxacéphalosporine (ou le cas échéant sur un mélange d'isomères bicyclooctène-2 et -3 d'un tel dérivé) de formule générale (I) dans laquelle $R_1$, $R_2$, $R_4$ et R'' sont définis comme précédemment en b), puis on élimine éventuellement les radicaux protecteurs de R, pour préparer un produit de formule générale:

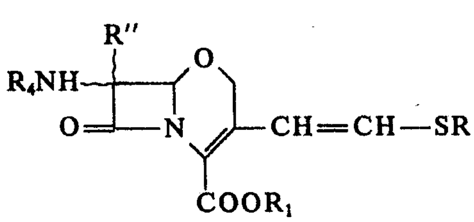 (XXVII)

dans laquelle $R_1$, $R_4$ et R″ sont définis comme pour les produits de formule générale (I) en b) et R est défini comme précédemment.

On opère alors selon l'un des schémas suivants:

2a) On prépare une amino-7 oxacéphalosporine de formule générale:

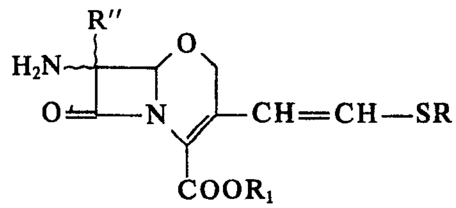

(XXVIII)

[dans laquelle R est défini comme précédemment, R″ est soit un atome d'hydrogène ou un radical méthoxy en 7$\alpha$, soit un atome d'hydrogène en 7$\beta$ et $R_1$ est un atome d'hydrogène ou un radical protecteur], à partir d'un produit de formule générale (XXVII) par élimination du radical $R_4$ (ou éventuellement élimination successive ou simultanée du radical $R_4$ et des autres groupements protecteurs).

Puis lorsque le radical R″ du produit de formule générale (XXVIII) est un atome d'hydrogène en 7$\beta$, et éventuellement lorsque le radical R″ du produit de formule générale (XXVIII) est un atome d'hydrogène en 7$\alpha$, on transforme le produit obtenu en le dérivé méthoxylé correspondant.

2b) Lorsque le radical $R_4$ est un radical facilement éliminable de formule générale (V), (VIII),(IX), (X) ou un radical bis(nitro-4 benzyl)phosphoryle, et R″ est un atome d'hydrogène en 7$\beta$ ou un atome d'hydrogène en 7$\alpha$ on méthoxyle le produit de formule générale (XXVII) pour obtenir le produit correspondant dans lequel R″ est un radical méthoxy en 7$\alpha$, puis on élimine le radical $R_4$ (ou éventuellement le radical $R_4$ et les autres radicaux protecteurs), pour obtenir une oxacéphalosporine de formule générale (XXVIII) dans laquelle R″ est un radical méthoxy en 7$\alpha$.

On prépare alors une oxacéphalosporine de formule générale (XXIII) par action d'un acide représenté par la formule générale

$$R'-OH \qquad \text{(XXIX)}$$

dans laquelle R′ est défini comme précédemment (étant entendu que lorsque R′ est un radical de formule générale (II), la fonction amine de ce radical est protégée) ou d'un dérivé réactif de cet acide, sur une amino-7 oxacéphalosporine de formule générale:

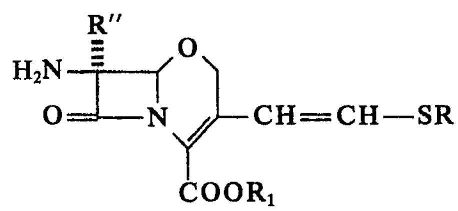

(XXVIIIa)

dans laquelle R est défini comme précédemment, R″ est un atome d'hydrogène ou un radical méthoxy en position 7$\alpha$ et $R_1$ représente un atome d'hydrogène ou un radical protecteur facilement éliminable, suivie de l'élimination des radicaux protecteurs.

D'un intérêt particulier sont les produits de formule générale (I) de forme bicyclooctène-2 dans laquelle $R_1$ est un radical benzhydryle, $R_4$ est un radical de formule générale (II) ou un radical trityle, R″ est un atome d'hydrogène en position 7$\alpha$ et $R_2$ est un radical de formule générale (IVa).

Et parmi ces produits, les produits de formule générale (I) de forme bicyclooctène-2 dans laquelle $R_1$ est un radical benzhydryle, $R_4$ est un radical de formule générale (II) [dans laquelle R° est un radical alcoyle] ou un radical trityle, R″ est un atome d'hydrogène en 7$\alpha$ et $R_2$ est un radical p.toluènesulfonyle.

Les exemples suivants, donnés à titre non limitatif, montrent comment l'invention peut être mise en pratique.

Dans ces exemples, les produits sont cités selon la nomenclature des Chemical Abstracts. Il est entendu qu'en l'absence de mention particulière les dérivés d'oxacéphalosporine qui sont cités présentent la stéréochimie donnée par la formule générale partielle:

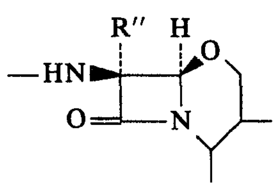

(XXX)

dans laquelle R″ est en position 7$\alpha$.

## Exemple 1

On dissout 2,51 g de benzhydryloxycarbonyl-2 (oxo-2 éthyl)-3 oxo-8 tritylamino-7 oxa-5 aza-1 bicyclo[4.2.0] octène-2 dans 20 cm3 de pyridine. A la solution obtenue, on ajoute 1,13 g de chlorure de tosyle et on maintient le mélange sous agitation à 20°C pendant 1 heure 25 minutes. La solution est versée dans 150 cm3 d'eau glacée, une gomme se dépose sur les parois du récipient, la phase aqueuse est décantée et on dissout la substance gommeuse dans 45 cm3 d'acétate d'éthyle. La solution organique est lavée par 2 fois 50 cm3 d'une solution 0,1 N d'acide chlorhydrique, 50 cm3 d'une solution à 5% de bicarbonate de sodium et 30 cm3 d'une solution demi-saturée de chlorure de sodium, séchée sur sulfate de sodium, filtrée et concentrée à sec à 30°C sous pression réduite (20 mm de mercure; 2,7 kPa). On recueille 2,4 g d'un produit brut marron constitué essentiellement de benzhydryloxycarbonyl-2 oxo-8 (tosyloxy-2vinyl)-3tritylamino-7oxa-5aza-1 bicyclo[4.2.0]octène-2, mélange des formes E et Z.

Spectre infra-rouge (KBr), bandes caractéristiques ($cm^{-1}$): 1790, 1725, 1595, 1490, 1450, 1380, 1190, 1180, 745, 700.

Le benzhydryloxycarbonyl-2 (oxo-2 éthyl)-3 oxo-8 tritylamino-7 oxa-5 aza-1 bicyclo[4.2.0] octène-2 peut être préparé de la manière suivante:

Une solution de 3,0 g de benzhydryloxycarbonyl-2 (diméthylamino-2 vinyl)-3 oxo-8 tritylamino-7 oxa-5 aza-1 bicyclo[4.2.0] octène-2, forme E, dans 100 cm3 d'acétate d'éthyle est agitée vigoureusement pendant 1 heure 30 à 20°C en présence de 45 cm3 d'une solution d'acide chlorhydrique 1N. Le mélange est décanté et la phase organique est lavée par 50 cm3 d'une solution à 5% de bicarbonate de sodium puis par 50 cm3 d'une solution demi-saturée de chlorure de sodium. La phase acétate d'éthyle est séchée sur sulfate de sodium, fitrée et concentrée à sec à 30°C sous pression réduite (20 mm de mercure; 2,7 kPa). On obtient 2,52 g d'un produit brut constitué principalement de benzhydrdryloxycarbonyl-2 (oxo-2 éthyl)-3 oxo-8 tritylamino-7 oxa-5 aza-1 bicyclo[4.2.0] octène-2 sous la forme d'une meringue marron clair.

Spectre infra-rouge (KBr), bandes caractéristiques ($cm^{-1}$): 1790, 1720, 1600, 1495, 1450, 1220, 750, 700.

Spectre de RMN du proton (350 MHz, $CDCl_3$, $\delta$ en ppm, J en Hz): 3,37 et

3,50 (2d, J = 16, 2H, $-C\underline{H}_2-$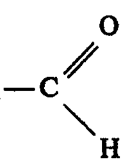);

3,81 (d, J = 3,5, 1H, H en 6); 3,92 et 4,12 (2d, J = 18, 2H, $-CH_2-O-$); 4,35 (dd, J = 3,5 et 9, 1H, H en 7); 6,80 (s, 1H, $-COO-CH(C_6H_5)_2$);

9,49 (s, 1H, —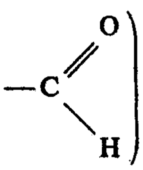).

Le benzhydryloxycarbonyl-2 (diméthylamino-2 vinyl)-3 oxo-8 tritylamino-7 oxa-5 aza-1 bicyclo[4.2.0] octène-2, forme E peut être obtenu de la mainière suivante: On chauffe à 80°C, sous azote, une solution de 4,25 g de benzhydryloxycarbonyl-2 méthyl-3 oxo-8 tritylamino-7 oxa-5 aza-1 bicyclo[4.2.0] octène-2 dans 20 cm3 de diméthylformamide. On ajoute, goutte à goutte en 7 minutes, dans la solution maintenue sous agitation à 80°C, 1,55 cm3 de t.butoxy bis-diméthylaminométhane. L'addition terminée, le mélange est conservé sous agitation à 80°C pendant 17 minutes. La solution est diluée par 150 cm3 d'acétate d'éthyle, la phase organique est lavée par trois fois 60 cm3 d'eau distillée et 60 cm3 d'une solution demi-saturée de chlorure de sodium, séchée sur sulfate de sodium, filtrée et concentrée à sec à 30°C sous pression réduite (20 mm de mercure; 2,7 kPa). La résidu est trituré dans 150 cm3 d'éther éthylique, la suspension obtenue est filtrée et le filtrat est concentré à sec à 30°C sous pression réduite (20 mm de mercure; 2,7 kPa). On obtient 3,14 g d'un produit brut constitué principalement de benzhydryloxycarbonyl-2 (diméthylamino-2 vinyl)-3 oxo-8 tritylamino-7 oxa-5 aza-1 bicyclo[4.2.0] octène-2, forme E utilisable sans purification supplémentaire.

Spectre infra-rouge (KBr), bandes caractéristiques ($cm^{-1}$): 1780, 1660, 1615, 1490, 1450, 745, 700.

Spectre de RMN du proton (350 MHz, $CDCl_3$ $\delta$ en ppm, J en Hz): 2,77 (s, 6H, $-N(CH_3)_2$); 3,71 (d, J = 3,5 1H, H en 6); 4,12 et 4,53 (2d, J = 17, 2H, $-CH_2-O-$); 4,26 (mf, 1H, H en 7); 6,24 et 6,40 (2d, J = 13, 2H, $-CH=CH-$); 6,81 (s, 1H, $-COO-CH(C_6H_5)_2$).

7,74 g de benzhydryloxycarbonyl-2 méthyl-3 oxo-8 tritylamino-7 oxa-5 aza-1 bicyclo[4.2.0] octène-2 sont préparés selon un schéma de synthèse décrit dans le brevet américain 4 108 992 dans lequel on remplace le glyoxylate de t-butyle par le glyoxylate de benzyhdryle préparé selon le brevet français 1 495 047.

L'oxacéphalosporine attendue est obtenue sous la forme d'un solide blanc à partir de 13,2 g de tritylamino-3 (propyne-2 yloxy)-4 oxo-2 azétidine.

Spectre infra-rouge (KBr), bandes caractéristiques ($cm^{-1}$): 3340, 1780, 1715, 1620, 1595, 1585, 1490, 1450, 1220, 745, 700.

Spectre de RMN du proton (350 MHz, $CDCl_3$, $\delta$ en ppm, J en Hz): 1,90 (s, 3H, $-CH_3$); 3,75 (d, J = 3,5 1H, H en 6); 3,87 et 4,08 (2d, J = 18, 2H, $-CH_2-O-$); 4,30 (d, J = 3,5, 1H, H en 7); 6,85 (s, 1H, $-COO-CH(C_6H_5)_2$; 7,15 à 7,4 (mt, 26H, aromatiques et $-HN-C(C_6H_5(_3)$.

Exemple 2

On ajoute en 10 minutes 0,27 cm3 de triéthylamine à une solution refroidie à $-5°C$ de 1,00 g de benzhydryloxycarbonyl-2 [méthoxyimino-2 (tritylamino-2 thiazolyl-4)-2 acétamido]-7 oxo-8 (oxo-2 éthyl)-3 oxa-5 aza-1 bicyclo[4.2.0] octène -2, isomère syn et de 0,35 g de chlorure de tosyle dans 20 cm3 de dichlorométhane. On laisse sous agitation à $-5°C$ pendant 10 minutes puis on laisse remonter à 20°C. Le mélange est alors agité pendant 30 minutes. Le solvant est évaporé sous pression réduite (350 mm de mercure, 47 kPa). On obtient ainsi une huile marron qui se solidifie par agitation avec 100 cm3 d'éther. Le solide est séparé sur filtre, et séché. On obtient 0,38 g de benzhydryloxycarbonyl-2 [méthoxy-imino-2 (tritylamino-2 thiazolyl-4)-2 acétamido]-7 oxo-8 (tosyloxy-2 vinyl)-3 oxa-5 aza-1 bicyclo[4.2.0] octène-2 isomère syn, forme E, sous la forme d'une poudre de couleur crème. Le filtrat est concentré jusqu'à un volume résiduel de 5 cm3, et on ajoute 100 cm3 d'éther isopropylique. Un solide jaune pâle se sépare. On l'isole sur filtre et obtient 0,11 g d'une deuxième fraction de benzyhdryloxycarbonyl-2[méthoxyimino-2 (tritylamino-2 thiazolyl-4)-2 acétamido]-7 oxo-8 (tosyloxy-2 vinyl)-3 oxa-5 aza-1 bicyclo[4.2.0] octène-2,lisomère syn, forme E.

Spectre infra-rouge (KBr), bandes caractéristiques ($cm^{-1}$) 3400, 1800, 1720, 1685, 1600, 1520, 1495, 1450, 1380, 1195, 1180, 1070, 815, 755, 700.

Spectre de RMN du proton (250 MHz, $CDCl_3$, $\delta$ en ppm, J en Hz) 2,44 (s, 3H, $CH_3$ tosyl); 4,08 (s, 3H, $=NOCH_3$); 4,44 (D, J = 16,25, 1H, $-O-CH_2-$); 4,64 (D, J = 16,25, 1H, $-O-CH_2-$); 5,12 (D, J = 3,75, 1H, $-H$ en 6); 5,79 (DD, J = 8 et 3,75, $-1H$, $-H$ en 7); 6,72 (D, J = 8, 1H, $-CONH-$); 6,77 (s, 1H, $-H$ du thiazole); 6,82 (D, J = 12,5, 1H, $-CH=CH-$); 6,83 (s, 1H, $-COOCH(C_6H_5)_2$); 7,03 (Mf, 1H, $(C_6H_5)_3$ CNH ·); 7,17 (D, J = 12,5, 1H, $-CH=CH-$); 7,20 à 7,55 (Mf, 27H aromatiques); 7,75 (D, J = 8, 2H ortho du tosyle).

Le benzhyaryloxycarbonyl-2 [methoxyimino-2 (tritylamino-2 thiazolyl-4)-2 acétamido]-7 oxo-8 (oxo-8 éthyl)-3 oxa-5 aza-1 bicyclo[4.2.0] octène-2, isomère syn peut être obtenu de la manière suivante:

Une solution de 1,05 g de benzhydryloxycarbonyl-2 (diméthylamino-2 vinyl)-3 [méthoxyimino-2 (tritylamino-2 thiazolyl-4)-2 acétamido]-7 oxo-8 oxa-5 aza-1 bicyclo [4.2.0] octène-2, isomère syn, forme E dans 10 cm3 d'acétate d'éthyle est agitée vigoureusement pendant 1 heure à 20°C en présence de 5 cm3 d'une solution 1N d'acide chlorhydrique. Le mélange est décanté et la phase organique est lavée par 10 cm3 d'une solution saturée de bicarbonate de sodium, 10 cm3 d'eau distillée et 10 cm3 d'une solution saturée de chlorure de sodium. La phase d'acétate d'éthyle est séchée sur sulfate de magnésium anhydre, filtrée et concentrée à sec à 30°C sous pression réduite (20 mm de mercure; 2,7 kPa). On obtient 1,00 g de benzhydryloxycarbonyl-2[méthoxyimino-2 (tritylamino-2 thiazolyl-4)-2 acétamido]-7 oxo-8 (oxo-2 éthyl)-3 oxa-5 aza-1 bicyclo[4.2.0] octène-2, isomère syn sous la forme d'une meringue jaune.

Spectre infra-rouge (KBr), bandes caractéristiques ($cm^{-1}$) 3400, 1795, 1725, 1685, 1525, 1495, 1450, 1035, 755, 700.

Spectre de masse: pic moléculaire = 817.

Specte de RMN du proton (250 MHz, $CDCl_3$, $\delta$ en ppm, J en Hz) 3,52 (D, J = 16,25, 1H, $-CH_2CHO$); 3,62 (D, J = 16,25, 1H, $-CH_2-CHO$); 4,07 (s, 3H, $=NOCH_3$); 4,35 (s, 2H, $-CH_2O-$); 5,17 (D, J = 3,75, 1H, $-H$ en 6); 5,82 (DD, J = 10 et 3,75, 1H, $-H$ en 7); 6,76 (D, J = 10, 1H, $-CONH-$); 6,80 (s, 1H, $-H$ du thiazole); 6,85 (s, 1H, $-COOCH(C_6H_5)_2$; 7,13 (mf, 1H, $(C_6H_5)_3$ CNH$-$); 7,20 à 7,55 (Mf, 25H, aromatiques); 9,60 (s, 1H, $-CHO$).

Le benzhydryloxycarbonyl-2 (diméthylamino-2 vinyl)-3 [méthoxyimino-2 (tritylamino-2 thiazolyl-4)-2 acétamido]-7 oxo-8 oxa-5 aza-1 bicyclo[4.2.0] octène-2, isomère syn, forme E peut être obtenu de la manière suivante:

On chauffe à 80°C sous azote, une solution de 0,79 g de benzhydroyloxycarbonyl-2 [méthoxyimino-2 (tritylamino-2 thiazolyl-4)-2 acétamido]-7 méthyl-3 oxo-8 oxa-5 aza-1 bicyclo [4.2.0] octène-2, isomère syn, dans 5 cm3 de diméthylformamide. On ajoute goutte à goutte en 6 minutes, dans la solution maintenue sous agitation à 80°C, 0,2 cm3 de t.butoxy bis-diméthylaminométhane. L'addition terminée, le mélange est conservé sous agitation à 80°C pendant 20 minutes. La solution est diluée par 25 cm3 d'acétate d'éthyle, la phase organique est lavée par trois fois 25 cm3 d'eau distillée et 25 cm3 d'une solution saturée de chlorure de sodium, séchée sur sulfate de magésium, filtrée et concentrée à sec à 40°C sous pression réduite (20 mm de mercure; 2,7 kPa). On obtient 0,71 g d'un produit brut constitué principalement de benzhydryloxycarbonyl-2 (diméthylamino-2 vinyl)-3[méthoxyimino-2 (tritylamino-2 thiazolyl-4)-2 acétamido]-7 oxo-8 oxa-5 aza-1 bicyclo [4.2.0] octène-2, isomère syn, forme E.

Un échantillon (0,4 g) est purifié par chromatographie sur une colonne de silice (0,04—0,06) (diamètre de la colonne: 2,2 cm; hauteur: 20 cm) en éluant sous 50 kPa avec un mélange de cyclohexane et d'acétate d'éthyle (40—60 en volumes) et en recueillant des fractions de 40 cm3. Les fractions 9 à 15 sont rassemblées et concentrées à sec sous pression réduite (30 mm de mercure; 4 kPa) à 40°C. On obtient 0,020 g d'une poudre jaune du produit pur.

Spectre de masse: pic moléculaire = 844.

Spectre de RMN du proton (350 MHz, $CDCl_3$, $\delta$ en ppm, J en Hz) 2,86 (s, 6H, $-N(CH_3)_2$); 4,07 (s, 3H, $=NOCH_3$); 4,61 et 4,76 (2D, J = 18, 2H, $-CH_2O-$); 5,10 (D, J = 3,5, 1H, H en 6); 5,69 (DD, J = 3,5 et 9, 1H, H en 7); 6,36 et 6,54 (2D, J = 14, 2H, $-CH=CH-$); 6,67 (D, J = 9, 1H, $-CONH-$); 6,82 (D, 1H, H thiazole); 6,86 (s, 1H, $-CO_2CH(C_6H_5)_2$); 7,01 (s, 1H, $-NH\ C(C_6H_5)_3$); 7,15 à 7,65 (m, 25H).

Spectre infra-rouge (KBr), bandes caractéristiques ($cm^{-1}$) 1780, 1685, 1615, 1525, 1490, 1445, 1120, 1030, 740, 695.

Le benzydryloxycarbonyl-2 [méthoxyimino-2 (tritylamino-2 thiazolyl-4)-2 acétamido]-7 méthyl-3 oxo-8 oxa-5 aza-1 bicyclo[4.2.0] octène-2, isomère syn, peut être préparé de la manière suivante:

Une solution de 35 g de tosylate d'amino-7 benzhydryloxycarbonyl-2 méthyl-3 oxo-8 oxa-5 aza-1 bicyclo[4.2.0] octène-2 dans 50 cm3 d'acétate d'éthyle est lavée par 15 cm3 d'une solution 1N de bicarbonate de sodium et 50 cm3 d'au distillée puis séchée sur sulfate de magésium. Après filtration, le solvant est évaporé sous pression réduite (20 mm de mercure; 2,7 kPa) à 40°C. On obtient 2,33 g d'amino-7 benzydryloxycarbonyl-2 méthyl-3 oxo-8 oxa-5 aza-1 bicyclo[4.2.0] octène-2 sous forme d'une meringue jaune pâle.

Spectre de masse: pic moléculaire = 364.

Spectre infra-rouge (KBr), bandes caractéristiques ($cm^{-1}$) 3400, 3340, 1780, 1720, 1640, 1495, 1445, 1230, 1110, 1060, 745, 700.

Spectre de RMN du proton (350 MHz, $CDCl_3$, $\delta$ en ppm, J en Hz) 2,03 (s, 3H, $-CH_3$); 4,32 (s, 2H, $-CH_2O-$); 4,47 CD, J = 3,5 1H, $-H$ en 7); 4,92 (D, J = 3,5, 1H, $-H$ en 6); 6,90 (s, 1H, $-CO_2CH(C_6H_5)_2$); 7,15 à 7,45 (m, 10H aromatiques).

A une solution de 2,3 g d'amino-7 benzhydryloxycarbonyl-2 méthyl-3 oxo-8 oxa-5 aza-1 bicyclo[4.2.0] octène-2 et de 2,79 g d'acide méthoxyimino-2 (tritylamino-2 thiazolyl-4)-2 acétique, isomère syn, dans 50 cm3 de chlorure de méthylène, on ajoute 1,56 g de N,N'-dicyclohexylcarbodiimide et 0,001 g de N,N-diméthylamino-4 pyridine en solution dans 40 cm3 de chlorure de méthylène en 15 minutes. Le mélange est maintenu sous agitation à 0°C pendant 2 heures. Il est alors tranféré dans une ampoule à décanter, puis lavé successivement par 50 cm3 d'une solution 0,1 N d'acide chlorhydrique, 50 cm3 d'eau distillée, 50 cm3 d'une solution demi-saturée de bicarbonate de sodium puis 50 cm3 d'eau distillée. La phase chlorométhylénique est séchée sur sulfate de magnésium anhydre. Le séchant filtré, la solution est concentrée à sec sous pression réduite (100 mm de mercure; 13,5 kPa). Le résidu (5,6 g) est purifié par chromatographie sur une colonne de silice (0,04—0,06) (diamètre de la colonne : 5,6 cm; hauteur: 26 cm) en éluant 50 kPa avec un mélange de cyclohexane et d'acétate d'éthyle (50—50 en volumes) et en recueillant des fractions de 120 cm3. Les fractions 8 à 20 sont rassemblées et concentrées à sec sous pression réduite (30 mm de mercure; 4 kPa) à 40°C. On obtient 4,02 g d'une meringue beige du benzhydryloxycarbonyl-2 [méthoxyimino-2 (tritylamino-2 thiazolyl-4)-2 acétamido)-7 méthyl-3 oxo-8 oxa-5 aza-1 bicyclo[4.2.0] octène-2, isomère syn, pur.

Spectre de masse: pic moléculaire = 789.

Spectre infra-rouge (KBr), bandes caractéristiques ($cm^{-1}$): 2820, 1790, 1745, 1580, 1530, 1495, 1450, 1165, 1040, 750, 700.

Spectre de RMN du proton (350 MHz, $CDCl_3$, $\delta$ en ppm, J en Hz) 2,04 (s, 3H, $-CH_3$); 4,07 (s, 3H, $=N-OCH_3$); 4,32 (s, 2H, $-CH_2O-$); 5,12 (D, J = 3,5, 1H, $-H$ en 6); 5,75 (DD, J = 3,5 et 9, 1H, $-H$ en 7); 6,68 (D, J = 9, 1H, $-CONH-$); 6,79 (s, 1H, H thiazole); 6,89 (s, 1H, $-CO_2CH(C_6H_5)_2$; 7,00 (s large, 1H, $-NH\ C(C_6H_5)_3$); 7,15 à 7,55 (m, 25H aromatiques).

Le tosylate d'amino-7 benzhydryloxycarbonyl-2 méthyl-3 oxo-8 oxa-5 aza-1 bicyclo[4.2.0] octène-2 peut être obtenu de la manière suivante:

On agite à 40°C pendant 30 minutes une solution de 6,07 g de benzhydryloxycarbonyl-2 méthyl-3 oxo-8 tritylamino-7 oxa-5 aza-1 bicyclo[4.2.0] octène-2 et 1,9 g d'acide p.toluènesulfonique hydraté dans 100 cm3 d'acétone. Le mélange est refroidi à 0°C et un solide blanc précipite. On essore et on lave le gâteau par deux fois 5 cm3 d'acétone. On obtient ainsi 3,17 g de tosylate d'amino-7 benzhydryloxycarbonyl-2 méthyl-3 oxo-8 oxa-5 aza-1 bicyclo[4.2.0] octène-2.

Le filtrat acétonique est concentré à sec sous pression réduite (20 mm de mercure; 2,7 kPa). Le résidu est trituré dans deux fois 100 cm3 d'éther éthylique. L'éther est décanté et le solide obtenu est repris dans 20 cm3 d'acétate d'éthyle. On filtre et on obtient une deuxième fraction de 0,72 g de tosylate d'amino-7 benzhydryloxycarbonyl-2 méthyl-3 oxo-8 oxa-5 aza-1 bicyclo[4.2.0] octène-2.

Spectre infra-rouge ($CHBr_3$), bandes caractéristiques ($cm^{-1}$) 3300, 2400, 1800, 1720, 1500, 1455, 1225, 820, 760, 745, 570.

Spectre de RMN du proton (350 MHz, $CDCl_3$, $\delta$ en ppm J en Hz)1,73 (s, 3H, $-CH_3$); 2,15 (s, 3H, $-CH_3$ (APTS) ); 3,98 (D, J = 19, 1H, $-CH_2O-$); 4,11 (D, J = 19, 1H, $-CH_2O-$); 4,78 (D, J = 2,5, 1H, $-H$ en 7); 4,86 (D, J = 2,5, 1H, $-H$ en 6); 6,87 (s, 1 H, $-COOCH(C_6H_5)_2$); 6,96 (D, J = 7,5, 1H, $-H$ en ortho du méthyle, APTS); 7,10 à 7,60 (Mt, 10H aromatiques); 7,74 (D, J = 7,5, 1H, $-H$ en ortho du $SO_3H$, APTS) ; 8,60 (Mf, 3H, $-NH_3+$).

Exemple de reference 1

Le produit de l'exemple 1 peut être utilisé de la manière suivante:

A une solution de 2,4 g de benzhydryloxycarbonyl-2 oxo-8 (tosyloxy-2 vinyl)-3 tritylamino-7 oxa-5 aza-1 bicyclo[4.2.0] octène-2 mélange des formes E et Z, dans 15 cm3 de diméthylformamide, on ajoute 0,28 g de sel de sodium de mercapto-5 méthyl-2 thiadiazole-1,3,4. Le mélange est agité à 20° C pendant 2 heures, puis est dilué par 50 cm3 d'acétate d'éthyle. La solution est lavée par 5 fois 50 cm3 d'eau distillée et 50 cm3 d'une solution demi-saturée de chlorure de sodium, puis séchée sur sulface de sodium. On filtre et concentre à sec à 20°C sous pression réduite (20 mm de mercure; 2,7 kPa). Le résidu (2,1 g) est chromatographié sur une colonne de gel de silice Merck (0,04—0,06) (diamètre de la colonne: 4,1 cm, hauteur: 20 cm). On élue par 1 litre d'un mélange de cyclohexane-acétate d'éthyle 70—30 (en volumes) sous une pression de 50 kPa en recueillant des fractions de 60 cm3. Les fractions 9 et 10 sont concentrée à sec sous pression réduite (30 mm de mercure 4 kPa) à 30° C. On obtient 0,13 g de benzhydryloxycarbonyl-2 [(méthyl-2 thiadiazol-1,3,4 yl-5) thio-2 vinyl]-3 oxo-8 tritylamino-7 oxa-5 aza-1 bicyclo[4.2.0] octène-2, forme E sous la forme d'une meringue jaune pâle.

Spectre infra-rouge (KBr), bandes caractéristiques ($cm^{-1}$): 1790, 1720, 1490, 1450, 1210, 745, 700.

Spectre de RMN du proton (350 MHz, $CDCl_3$, $\delta$ en ppm, J en Hz): 2,74 (s, 3H, $-CH_3$); 3,76 (d, J = 3,5, 1H, H en 6); 4,16 et 4,62 (2d, J = 18, 2H, $-CH_2-O-$); 4,37 (d, J = 3,5, 1H, H en 7); 6,84 (s, 1H, $-COO-CH(C_6H_5)_2$); 6,96 (d, J = 17, 1H, $-CH=CH-S-$).

On dissout 0,112 g de benzhydryloxycarbonyl-2 [(méthyl-2 thiadiazol-1,3,4 yl-5) thio-2 vinyl]-3 oxo-8 tritylamino-7 oxa-5 aza-1 bicyclo[4.2.0] octène-2, forme E dans 1,4 cm3 d'acétone et on ajoute à la solution 0,029 g d'acide p.toluènesulfonique monohydraté. La solution est portée au reflux pendant 45 minutes pendant lesquelles des cristaux se développent sur les parois du récipient. La suspension est filtrée et le filtrat est versé dans 10 cm3 d'une solution à 1% de bicarbonate de sodium. Le mélange est extrait par 2 fois 5 cm3 d'acétate d'éthyle, la phase organique est séchée sur sulfate de sodium, fitrée et concentrée à sec à 30° C sous pression réduite (20 mm de mercure; 2,7 kPa). On recueille ainsi 0,1 g d'un solide brut marron cinstitué essentiellement d'amino-7 benzhydryloxycarbonyl-2 [(méthyl-2 thiadiazol-1,3,4 yl-5) thio-2 vinyl]-3 oxo-8 oxa-5 aza-1 bicyclo[4.2.0] octène-2, forme Z.

Rf = 0,20 [chromatoplaque de silicagel, éluant: cyclohexane-acétate d'éthyle 80—20 (en volumes)].

A une solution de 0,1 g d'amino-7 benzhydryloxycarbonyl-2 [(méthyl-2 thiadiazol-1,3,4 yl-5) thio-2 vinyl]-3 oxo-8 oxa-5 aza-1 bicyclo[4.2.0] octène-2, forme E dans 20 cm3 de dichlorométhane, on ajoute 0,089 g d'acide méthoxyimino-2 (tritylamino-2 thiazolyl-4)-2 acétique, isomère syn, 0,041 g de N,N'-dicyclohexylcarbodiimide et 0,001 g de N,N-diméthylamino-4 pyridine. Le mélange est agité à 20° C pendant 1 heure 30. On ajoute alors 0,1 cm3 d'acide acétique; on élimine un léger insoluble par filtration, concentre à sec le filtrat à 30° C sous pression réduite (100 mm de mercure) et dissout le résidu dans 5 cm3 d'acétate d'éthyle. La solution est lavée par 2 fois 2,5 cm3 d'acide chlorhydrique 0,1 N, puis par 5 cm3 d'une solution à 1% de bicarbonate de sodium et par 5 cm3 d'eau distillée. On sèche sur sulfate de sodium, filtre et concentre à sec à 30° C sous 20 mm de mercure (2,7 kPa). Le résidu (0,12 g) est fixé sur 0,25 g de gel de silice Merck (0,05—0,2) et la poudre obtenue est déposée sur une colonne de 10 g de gel de silice (diamètre de la colonne: 1 cm). On élue successivement par 70 cm3 d'un mélange cyclohexane — acétate d'thyle 50-50 (en volumes), 30 cm3 d'un mélange 40—60 et 30 cm3 d'un mélange 20—80 en recueillant des fractions de 2,5 cm3. On concentre à sec à 20° C sous pression réduite (20 mm de mercure; 2,7 kPa) les fractions 22 à 40 et recueille 0,023 g de benzhydryloxycarbonyl-2 [méthoxyimino-2 (tritylamino-2 thiazolyl-4)-2 acétamido]-7 [(méthyl-2 thiadiazol-1,3,4 yl-5) thio-2 vinyl]-3 oxo-8, oxa-5 aza-1 bicyclo[4.2.0] octène-2 isomère syn, forme E sous la forme d'une meringue organée.

Spectre infra-rouge (KBr), bandes caractéristiques ($cm^{-1}$): 1795, 1720, 1685, 1520, 1495, 1450, 1210, 1045, 750, 700.

Spectre de RMN du proton (350 MHz, $CDCl_3$, $\delta$ en ppm, J en Hz): 2,74 (s, 3H, $-CH_3$); 4,07 (s, 3H, $=N-O-CH_3$); 4,59 et 4,86 (2d, J = 18, 2H, $-CH_2-O-$); 5,13 (d, J = 3,5, 1H, —H en 6); 5,81 (dd, J = 3,5 et 9, 1H, —H en 7); 6,78 (s, 1H, —H du thiazole); 6,79 (d, J = 9, 1H, —CO—NH—); 6,88 (s, 1H, $-COO-CH(C_6H_5)_2$); 7,03 (mf, 1H, $-NH-C(C_6H_5)_3$); 7,14 et 7,69 (2d, J = 17, 2H, $-CH=CH-S-$).

A une solution de 0,023 g de benzhydryloxycarbonyl-2 [méthoxyimino-2 (tritylamino-2 thiazolyl4)-2 acétamido]-7 [(méthyl-2 thiadiazol-1,3,4 yl-5) thio-2 vinyl]-3 oxo-8 oxa-5 aza-1 bicyclo [4.2.0] octène-2, isomère syn, forme E, dans 1 cm3 d'acide formique, on ajoute 0,5 cm3 d'eau distillée et chauffe le mélange à 50°C sous agitation pendant 20 minutes. Après refroidissement, on filtre l'insoluble et on concentre à sec à 30°C sous pression réduite (20 mm de mercure; 2,7 kPa). On reprend le résidu par 2 fois 15 cm3 d'éthanol en concentrant à sec à chaque fois à 20°C sous 20 mm de mercure (2,7 kPa) et triture le résidu dans 20 cm3 d'éther éthylique. On obtient après filtration 0,010 g d'[(amino-2 thiazolyl - 4)-2 méthoxyimino-2 acétamido]-7 carboxy-2 [(méthyl-2 thiadiazol1,3,4 yl-5) thio-2 vinyl]-3 oxo-8 oxa-5 aza-1 bicyclo[4.2.0] octène-2, isomère syn, forme E sous la forme d'une poudre jaune.

Spectre infra-rouge (KBr), bandes caractéristiques ($cm^{-1}$): 1785, 1670, 1620, 1530, 1040.

Spectre de RMN du proton 350 MHz, DMSO $d_6$, $\delta$ en ppm, J en Hz): 2,70 (s, $-CH_3$); 3,83 (s, $=N-OCH_3$); 4,50 et 4,78 (2d, J = 18, $-CH_2-O-$); 5,14 (d, J = 3,5 —H en 6); 5,45 (mf, —H en 7); 6,75 (s, —H du thiazole); 7,10 à 7,70 (mt, $-NH_2$ et $-CH=CH-S-$); 9,34 (d, J = 9, —CO—NH—).

Exemple de reference 2

Le produit de l'exemple 2 peut être utilisé de la manière suivante:

Une solution de 0,53 g de benzhydryloxycarbonyl-2 [méthoxyimino-2 (tritylamino-2 thiazolyl4)-2 acétamido]-7 oxo-8 (tosyloxy-2 vinyl)-3 oxa 5 aza 1 bicyclo[4.2.0] octène-2, isomère syn, forme E, et de 0,139 g de [(diméthoxy-2,2 éthyl)-4 tétrahydro-1,4,5,6 dioxo-5,6 triazine-1,2,4 yl-3] thiolate de sodium dans 12 cm3 de diméthylformamide est portée à 40° C sous agitation pendant 6 heures. Le mlange est transféré dans une ampoule à décanter contenant 30 cm3 d'acétate d'thyle et 50 cm3 d'eau distillée. Après décantation la phase organique est lavée par 3 fois 50 cm3 d'eau distillée puis séchée sur sulfate de magnésium anhydre. Le solvant est évaporé sous pression réduite (20 mm de mercure; 2,7 kPa) et le résidu (0,52 g) est purifié par chromatographie sur une colonne de gel de silice Merck (0,04—0,06) diamètre de la colonne: 2,2 cm, hauteur: 25,5 cm). On élue par un mélange de cyclohexane-acétate d'éthyle 15—85 (en volumes) sous une pression de 50 kPa en recueillant des fractions de 20 cm3.

Les fractions 10 à 28 sont concentrées à sec sous pression réduite (30 mm de mercure; 4 kPa) à 30° C. On obtient 0,20 g de benzhydryloxycarbonyl-2 [[[(diméthoxy-2,2 éthyl)-4 tétrahydro-1,4,5,6 dioxo-5,6 triazine-1,2,4 yl-3] thio-2 vinyl]]-3 [méthoxyimino-2 (tritylamino-2 thiazolyl-4)-2 acétamido]-7 oxo-8 oxa-5 aza-1 bicyclo[4.2.0] octène-2, isomère syn, forme E, sous la forme d'une meringue marron clair.

Spectre de RMN du proton (250 MHz, $CDCl_3$, $\delta$ en ppm, J en Hz) 3,42 (s, 6H, $(-OCH_3)_2$) 4,03 (AB limite J = 4,5 et 10, 2H, $> NCH_2-$); 4,06 (s, 3H, $=N-OCH_3$); 4,58 (D, J = 16,25, 1H, $-OCH_2-$); 4,65 (T, J = 5, 1H, $-CH(OCH_3)_2$); 4,84 (D, J = 16,25, 1H, $-O-CH_2-$); 5,15 (D, J = 3,15, 1H, —H en 6); 5,81 (DD, J 1 = 10 et 3,15, 1H, —H en 7); 6,73 (D, J = 16,25, 1H, $-CH=CH-$); 6,79 (s, 1H, —H du thiazole); 6,87 (s, 1H, $-COO\ CH(C_6H_5)_2$); 7,05 (Mf 1H, $(C_6H_5)_3CNH-$); 7,18 (D, J = 10, 1H, —CONH—); 7,20 à 7,55 (Mf, 25H aromatiques); 7,68 (D, J = 16,25, 1H, $-CH=CH-$); 10,09 (s large, 1H —NH— triazine).

On ajoute 0,74 g d'acide p.toluènesulfonique monohydraté à une solution de 0,20 g de benzhydryloxycarbonyl-2 [[[(diméthoxy-2,2 éthyl)-4 tétrahydro-1,4,5,6 dioxo-5,6 triazine1-2,4 yl-3] thio-2 vinyl]]-3 [méthoxyimino-2 (tritylamino-2 thiazolyl-4)-2 acétamido]-7 oxo-8 oxa-5 aza-1 bicyclo[4.2.0] octène 2, isomère syn, forme E, dans 5 cm3 d'acétonitrile à 50° C. Le mélange est maintenu à cette température pendant 30 minutes. Après refroidissement à 20° C, on filtre le précipité qui s'est développé au cours de la réaction.

Il est lavé par 1 cm3 d'acétonitrile, puis il est agité vigoureusement dans 5 cm3 d'eau distillée pendant 30 minutes. La suspension est filtrée, et la poudre marron obtenue est séchée sous pression réduite (10 mm de mercure; 1,33 kPa) donnant 20 mg de tosylate d'[amino-2 thiazolyl-4)-2 méthoxyimino-2 acétamido]-7 carboxy-2 [[[tétrahydro-1,4,5,6 dioxo-5,6 (oxo-2 éthyl)-4 triazine1,2,4 yl-3] thio-2 vinyl]]-3 oxo-8 oxa-5 aza-1 bicyclo[4.2.0] octène-2, isomère syn, forme E.

Spectre de RMN du proton (250 MHz, $CF_3CO_2D$, $\delta$ en ppm, J en Hz) 2,47 (s, 3H, $-CH_3$); 4,30 (s, 3H $=NOCH_3$); 5,17 (Mf, $> NCH_2-$); 5,45 (Mf, 1H, —H en 6); 5,91 (Mf, 1H, —H en 7); 7,39 (D, J = 8, 2H, H tosyle); 7,53 (s, 1H, —H du thiazole); 7,82 (D, J = 8, 2H, H tosyl); 9,76 (s, 1H, —CHO).

**Revendications pour les Etats contractants: BE, CH, DE, FR, GB, IT, LI, LU, NL, SE**

1. Dérivé d'oxacéphalosporine caractérisé en ce qu'il répond à la formule générale:

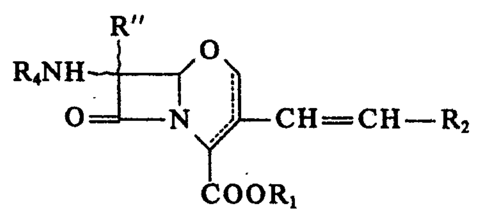

(I)

qui se présente sous forme bicyclooctène-2 ou -3 et dont le substituant en position -3 présente la stéréoisomérie E ou Z et dans laquelle

a) le symbole $R_1$ représente un atome d'hydrogène ou un radical protecteur d'acide facilement éliminable,

le symbole $R_4$ représente un atome d'hydrogène ou un radical de formule générale:

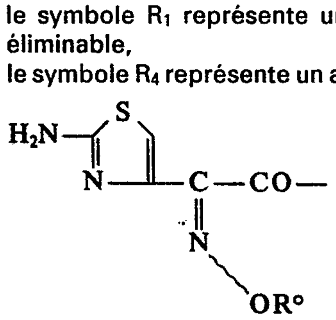

(II)

[dont la fonction amine est libre ou protégée et dans laquelle R° représente un atome d'hydrogène, un radical protecteur d'oxime, un radical alcoyle, vinyle, ou carboxyalcoyle libre ou protégé représenté par la formule générale:

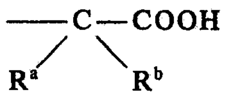 (III)

dans laquelle les radicaux $R^a$ et $R^b$ qui sont identiques ou différents rprésentent des atomes d'hydrogène ou des radicaux alcoyle, ou forment ensemble un radical alcoylène contenant 2 ou 3 atomes de carbone], ou $R_4$ représente un radical $\alpha$-carboxyarylacétyle dont le groupement carboxy peut être libre ou protégé et dans lequel aryle représente phényle (éventuellement substitué par un radical p.hydroxy libre ou protégé), ou thiényle-2 ou -3, et

le symbole R″ représente un atome d'hydrogène ou un radical méthoxy en position $7\alpha$, ou bien

b) le symbole $R_1$ représente un radical protecteur d'acide facilement éliminable, le symbole $R_4$ représente un radical faciliment éliminable, et

le symbole R″ représente un atome d'hydrogène ou un radical méthoxy en $7\alpha$, ou un atome d'hydrogène en $7\beta$ et,

le symbole $R_2$ représente un radical de formule générale:

$-O-SO_2R_3$ (IVa)

ou

$-O-COR'_3$ (IVb)

[dans lesquelles $R_3$ est un radical alcoyle droit ou ramifié contenant 1 à 4 atomes de carbone, trifluorométhyle, trichlorométhyle, ou phényle non substitué ou substitué par un atome d'halogène ou par un radical alcoyle ou nitro, et

$R'_3$ est défini comme $R_3$ ou représente un radical acylméthyle, acyl-2 éthyle, acyl-2 propyle, alcoyloxycarbonylméthyle, alcoyloxycarbonyl-2 éthyle ou alcoyloxycarbonyl-2 propyle], ou un atome d'halogène, étant entendu que les radicaux et protions alcoyles ou acyles cités ci-dessus sont (sauf mention spéciale) droits ou ramifißaes et contiennent 1 à 4 atomes de carbone,

sous ses formes E ou Z et leurs mélanges, et le cas échéant sous ses formes syn on anti et leurs mélanges.

2. Dérivé d'oxacéphalosporine selon la revendication 1, caractérisé en ce que $R_1$ et R″ étant définis comme en a) dans la revendication 1, $R_4$ représente un radical de formule générale:

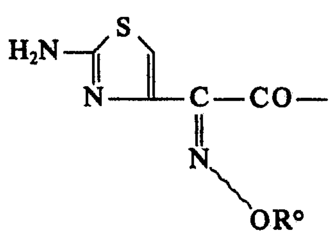 (II)

dans laquelle R° est défini selon la revendication 1, et dont la fonction amine est préalablement protégé par un groupement t.butoxycarbonyle, trichloro-2,2,2 éthoxycarbonyle, trichloracéthyle, chloracétyle, trityle, dibenzyle, benzyle, benzyoxycarbonyle, p.nitrobenzyloxycarbonyle, p.méthoxybenzyloxycarbonyle, formyle ou trifluoracétyle, et $R_2$ est défini comme dans la revendication 1.

3. Dérivé d'oxacéphalosporine selon la revendication 1 caractérisé en ce que, $R_1$ et R″ étant définis comme en a) dans la revendication 1, $R_4$ représente un radical de formule générale:

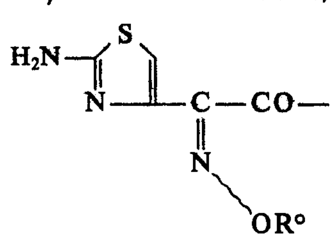 (II)

dont la fonction amine est libre ou protégé et dans laquelle R° représente un groupement trityle, tétrhydropyrannyle ou méthoxy-2 propyle-2 protecteur de la fonction oxime, et $R_2$ est défini comme dans la revendication 1.

4. Dérivé d'oxacéphalosporine selon la revendication 1 caractérisé en ce que, $R_1$ et R″ étant définis comme en a) dans la revendication 1, $R_4$ représente un radical de formule générale:

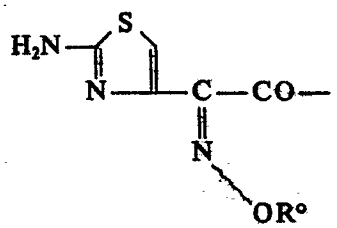 (II)

dont la fonction amine est libre ou protégée et dans laquelle R° représente un groupement carboxyalcoyle [de formule générale:

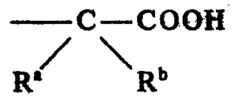 (III)

dans laquelle $R^a$ et $R^b$ sont définis comme dans la revendication 1] protégé par un radical choisi parmi méthoxyméthyle, t.butyle, benzhydryle, benzyle, nitrobenzyle et p.méthoxybenzyle, et $R_2$ est défini comme dans la revendication 1.

5. Dérivé d'oxacéphalosporine selon la revendication 1 caractérisé en ce que, $R_1$ et R″ étant définis comme en a) dans la revendication 1, $R_4$ représente un radical $\alpha$-carboxyarylacétyle dont le groupement carboxy est protégé par un radical choisi parmi méthoxy-méthyle, t.butyle, benzhydryle, benzyle, nitrobenzyle et p.méthoxybenzyle.

6. Dérivé d'oxacéphalosporine selon la revendication 1 caractérisé en ce que, $R_2$, $R_4$ et R″ étant définis comme dans la revendication 1, le radical facilement éliminable représenté par $R_1$ est choisi parmi les radicaux méthoxyméthyle, t.butyle, benzhydryle, benzyle, p.nitrobenzyle et p.méthoxybenzyle.

7. Dérivé d'oxacéphalosporine selon la revendication 1 caractérisé en ce que, $R_1$ et R″ étant définis comme en b) dans la revendication 1, $R_4$ représente un radical facilement éliminable choisi parmi:

1. benzhydryle ou trityle,
2. un radical acyle de formule générale:

$R_5CO-$ (V)

dans laquelle $R_5$ représente:

a) un atome d'hydrogène, un radical alcoyle contenant 1 à 7 atomes de carbone, méthyle substitué par 1 à 3 atomes d'halogène, alcényle contenant 3 à 7 atomes de carbone ou cyanométhyle,
b) un radical phényle (pouvant être jusqu'à 3 fois substitué par un atome d'halogène ou par des radicaux hydroxy, nitro, cyano, trifluorométhyle, alcoyle, ou alcoyloxy) ou un radical thiényle-2 ou -3
c) un radical de formule générale:

$R'_5YCH_2-$ (VIa)

dans laquelle $R'_5$ représente un radical défini en b) et Y représente un atome de soufre ou d'oxygène

d) un radical arylalcoyle de formule générale:

$R''_5CH_2-$ (VIb)

dans laquelle $R''_5$ représente un radical phényle (pouvant être jusqu'à 3 fois substitué par des radicaux hydroxy, alcoyle ou alcoyloxy) ou un radical hétérocyclyle choisi parmi thiényle-2 ou -3, furyle-2 ou -3 ou tétrazolyle-1,

3. un radical de formule générale:

$R_6OCO-$ (VII)

dans laquelle $R_6$ représente un radical alcoyle ramifié non substitué un radical alcoyle droit ou

ramifié portant un ou plusieurs substituants choisi parmi des atomes d'halogène ou des radicaux cyano, phényle ou phényle substitué (par un ou plusieurs atomes d'halogène ou radicaux alcoyle, alcoyloxy, nitro ou phényle), un radical triméthylsilyl-2 éthyle, un radical vinyle ou allyle ou un radical quinolyle,

4. un radical de formule générale:

$$R'_6-\underset{(O)_n}{\underset{|}{S}}- \quad (VIII) \qquad \text{ou} \qquad R'_6-Se- \quad (IX)$$

dans lesquelles le symbole $R'_6$ représente un radical alcoyle ou phényle ou phényle substitué par un ou plusieurs atomes d'halogène ou radicaux nitro ou alcoyle et n'est égal à 0 ou 1,

5. ou encore $R_4$ représente un radical bis(nitro-4 benzyl)phosphoryle,
6. ou bien $R_4NH$ est remplacé par un radical alcoylaminométhylèneamino ou par un radical de formule générale:

$$AR-C=N- \qquad (X)$$

dans laquelle Ar représente un groupe phényle éventuellement substitué par un ou plusieurs radicaux choisis parmi hydroxy, nitro, alcoyle ou alcoyloxy,

8. Un procédé de préparation d'un produit selon la revendication 1 pour lequel $R_1$, $R_2$, $R_4$ et R'' sont définis comme dans la revendication 1 à l'exception pur $R_4$ de représenter un atome d'hydrogène, caractérisé en ce que l'on fait agir soit un dérivé activé des acides $R_3SO_3H$ et $R'_3COOH$, choisi parmi:

$$\left.\begin{array}{l}(R_3SO_2)_2O\\ R_3SO_2Hal\\ (R'_3CO)_2O\\ \text{et} \quad R'_3COHal\end{array}\right\} \qquad (XI)$$

($R_3$ et $R'_3$ étant définis comme dans la revendication 1 et Hal représentant un atome d'halogène) soit un agent d'halogéation, sur un produit de formule générale:

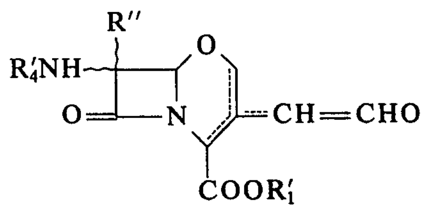

(XII)

(ou sur un mélange de ses isomères), qui se présente sous forme bicyclooctène-2 ou -3, ou oxoéthylidène-3 bicyclooctane, et dans laquelle $R'_1$ est un radical protecteur facilement éliminable, et soit $R'_4$ est défini comme $R_4$ dans la revendication 1 en a) à l'exception de représenter l'hydrogène étant entendu que lorsque $R'_4$ contient un radical amino ce dernier est protégé, et R'' est un atome d'hydrogène ou un radical méthoxy en $7\alpha$ soit $R'_4$ est défini comme $R_4$ dans la revendication 1 en b) et R'' est un atome d'hydrogène ou un radical méthoxy en position $7\alpha$ ou un atome d'hydrogène en position $7\beta$, puis on élimine éventuellement les radicaux protecteurs et sépare évenutellement les isomères du produit obtenu.

9. Un procédé de préparation d'un produit selon la revendication 1 pour lequel $R_1$, $R_2$ et R'' sont définis comme dans la revendication 1 en a) et $R_4$ est un atome d'hydrogène, caractérisé en ce que l'on élimine le radical $R_4$ ou éventuellement en ce que l'on élimine simultanément le radical $R_4$ et le radical $R_1$ d'un produit selon la revendication 1 pour lequel $R_1$ et $R_4$ sont définis comme dans la revendication 1 en b), puis on sépare éventuellement le produit obtenue en ses isomères.

10. Un procédé de préparation d'un produit selon la revendication 1 pour lequel $R_1$, $R_2$, $R_4$ et R'' sont définis comme dans la revendication 1 en a), à l'exception pour $R_4$ de représenter un atome d'hydrogène, caractérisé en ce que l'on acyle au moyen d'un acide représenté par la formule générale:

$$R_4-OH \qquad (XXI)$$

(dans laquelle $R_4$ est défini comme ci-dessus et dont le cas échéant la fonction amine est préalablement protégée) ou d'un de ses dérivés réactifs une oxacéphalosporine selon la revendication 1 pour

laquelle $R_4$ représente un atome d'hydrogène, puis on élimine éventuellement les radicaux protecteurs et sépare éventuellement le produit obtenu en ses isomères.

**Revendication pour l'Etat contractant: AT**

Procédé de préparation d'un dérivé d'oxacéphalosporine de formule générale:

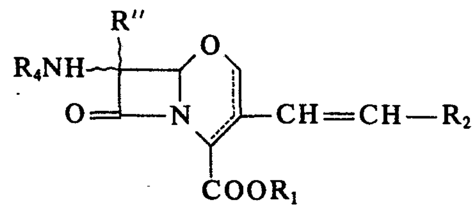

(I)

qui se présente sous forme bicyclooctène-2 ou -3 et dont le substituant en position -3 présente la stéréoisomérie E ou Z et dans laquelle

a) le symbole $R_1$ reprénte un atome d'hydrogène ou un radical protecteur d'acide facilement éliminable,
le symbole $R_4$ représente un atome d'hydrogène ou un radical de formule générale:

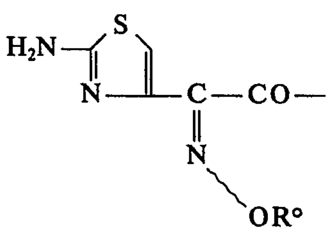

(II)

[dont la fonction amine est libre ou protégée et dans laquelle R° représente un atome d'hydrogène, un radical protecteur d'oxime, un radical alcoyle, vinyle, ou carboxyalcoyle, libre ou protégé représenté par la formule générale:

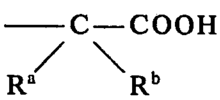

(III)

dans laquelle les radicaux $R^a$ et $R^b$ qui sont identiques ou différents représentent des atomes d'hydrogène ou des radicaux alcoyle, ou forment entsemble un radical alcoylène contenant 2 ou 3 atomes de carbone], ou $R_4$ représente un radical $\alpha$-carboxyarylacétyle dont le groupement carboxy peut être libre ou protégé et dans lequel aryle représente phényle (éventuellement substitué par un radical p.hydroxy libre ou protégé), ou thiényle-2 ou-3, et
le symbole R'' représente un atome d'hydrogène ou un radical méthoxy en position $7\alpha$, ou bien

b) le symbole $R_1$ représente un radical protecteur d'acide facilement éliminable, le symbole $R_4$ représente un radical facilement éliminable, et
le symbole R'' représente un atome d'hydrogène ou un radical méthoxy en $7\alpha$, ou un atome d'hydrogène en $7\beta$ et,
le symbole $R_2$ représente un radical de formule générale:

$$-O-SO_2R_3 \quad \text{(IVa)}$$

ou

$$-O-COR'_3 \quad \text{(IVb)}$$

[dans lesquelles $R_3$ est un radical alcoyle droit ou ramifié contenant 1 à 4 atomes de carbone, trifluorométhyle, trichlorométhyle, ou phényle, non substitué ou substitué par un atome d'halogène ou par un radical alcoyle ou nitro, et
$R'_3$ est défini comme $R_3$ ou représente un radical acylméthyle, acyl-2 éthyle, acyl-2 propyle, alcoyloxycarbonylméthyle, alcoyloxycarbonyl-2 éthyle ou alcoyloxycarbonyl-2 propyle], ou un atome d'halogène, étant entendu que les radicaux et protions alcoyles ou acyles cités ci-dessus sont (sauf mention spéciale) droits ou ramifiés et contiennent 1 à 4 atomes de carbone,

sous ses formes E ou Z et leurs mélanges, et le cas échéant sous ses formes syn on anti et leurs mélange, caractérisé en ce que

A — pour la préparation d'un produit dans la formule duquel $R_4$ est autre qu'un atome d'hydrogène, on fait agir soit un dérivé activé des acides $R_3SO_3H$ et $R'_3COOH$ choisi parmi:

$(R_3SO_2)_2O$
$R_3SO_2Hal$
$(R'_3CO)_2O$
et $R'_3COHal$ (XI)

($R_3$ et $R'_3$ étant définis comme ci-dessus et Hal représentant un atome d'halogène) soit un agent d'halogénation, sur un produit de formule générale:

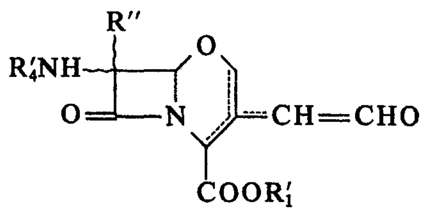

(XII)

(ou sur un mélange de ses isomères), qui se présente sous forme bicycloocténe-2- ou -3, ou oxoéthylidène-3 bicyclooctane, et dans laquelle $R'_1$ représente un radical protecteur facilement éliminable, et soit $R'_4$ est défini comme $R_4$ ci-dessus en a) à l'exception de représenter l'hydrogène, étant entendu que lorsque $R'_4$ contient un radical amino ce dernier est protégé, et R″ représente un atome d'hydrogène ou un radical méthoxy en $7\alpha$, soit $R'_4$ est défini comme $R_4$ en b), et R″ représente un atome d'hydrogène ou un radical méthoxy en position $7\alpha$ ou un atome d'hydrogène en position $7\beta$ puis on élimine éventuellement les radicaux protecteurs et sépare éventuellent les isomères du produit obtenu, puis

B — pour la préparation d'un produit dans la formule duquel $R_4$ représente un atome d'hydrogène, on élimine le radical $R_4$ ou éventuellement on élimine simultanément le radical $R_4$ et le radical $R_1$ d'un produit préparé selon A- et pour lequel $R_1$ et $R_4$ sont définis comme ci-dessus en b), puis on séparé éventuellement le produit obtenu en ses isomères, puis

C — pour la préparation d'un produit dans la formule duquel $R_4$ est autre qu'un atome d'hydrogène, on acyle au moyen d'un acide représenté par la formula générale:

$R_4{-}OH$ (XXI)

(dans laquelle $R_4$ est défini comme ci-dessus et dont le cas échéant la fonction amine est préalablment protégée) ou d'un de ses dérivés réactifs une oxacéphalosporine préparée selon B-, puis on élimine éventuellement les radicaux protecteurs et séparé éventuellement le produit obtenu en ses isomères.

**Patentansprüche für die Vertragsstaaten: BE, CH, DE, FR, GB, IT, LI, LU, NL, SE**

1. Oxacephalosporinderivat, dadurch gekennzeichnet, daß der allgemeinen Formel (I)

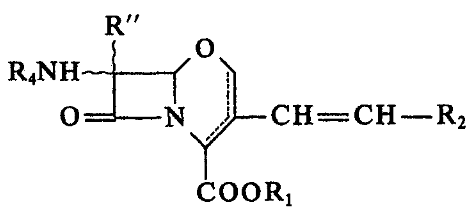

(I)

entspricht, welche unter der Bicyclooncten-2- oder 3-Form vorliegt und dessen Substituent in 3-Stellung die E- oder Z-Stereoisomerie aufweist und worin

a) das Symbol $R_1$ ein Wasserstoffatom oder einen leicht entfernbaren Säureschutzrest bedeutet, das Symbol $R_4$ ein Wasserstoffatom oder einen Rest der allgemeinen Formel (II)

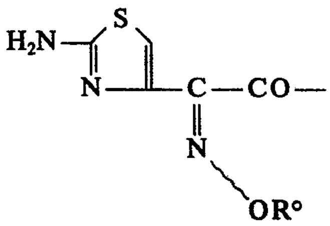 (II)

bedeutet,[dessen Aminfunktion frei oder geschützt ist und worin R° ein Wasserstoffatom, einen Oxim-Schutzrest, einen Alkyl-, Vinyl- oder Carboxylrest, frei oder geschützt, bedeutet, dargestellt durch die allgemeine Formel (III),

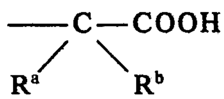 (III)

worin die Reste $R^a$ und $R^b$, welche identisch oder verschieden sind, Wasserstoffatome oder Alkylreste bedeuten oder zusammen einen Alkylenrest mit 2 oder 3 Kohlenstoffatomen bilden] oder $R_4$ bedeutet einen $\alpha$-Carboxyarylacetyl-Rest, dessen Carboxygruppe frei oder geschützt sein kann und worin Aryl Phenyl (gegebenenfalls substituiert durch einen freien oder geschützten p-Hydroxy-Rest) oder Thienyl-2 oder -3 bedeutet, und
das Symbol R'' ein Wasserstoffatom oder einen Methoxyrest in $7\alpha$-Stellung bedeutet oder

b) das Symbol $R_1$ bedeutet einen leicht entfernbaren Säureschutzrest, das Symbol $R_4$ bedeutet einen leicht entfernbaren Rest und das Symbol R'' bedeutet ein Wasserstoffatom oder einen Methoxyrest in $7\alpha$ oder ein Wasserstoffatom in $7\beta$, und
das Symbol $R_2$ bedeutet einen Rest der allgemeinen Formel (IVa) oder IVb)

$-\dot{O}-SO_2R_3$ (IVa)

oder

$-O-COR'_3$ (IVb)

[worin $R_3$ ein gerader oder verzweigter Alkylrest mit 1 bis 4 Kohlenstoffatomen, Trifluormethyl, Trichlormethyl oder Phenyl ist, nicht substituiert oder substituiert durch ein Halogenatom oder durch einen Alkyl- oder Nitrorest und $R'_3$ ist wie $R_3$ definiert oder bedeutet einen Acylmethyl-, 2-Acyläthyl-, 2-Acylpropyl-, Alkyloxycarbonylmethyl-, 2-Alkoxycarbonyl-äthyl- oder 2-Alkyloxycarbonyl-propyl-Rest] oder ein Halogenatom, wobei die oben erwähnten Alkyl- oder Acylreste und -teile (wenn nichts anderes angegeben ist) gerade oder verzweigt sind und 1 bis 4 Kohlenstoffatome enthalten,
unter seinen E- oder Z-Formen und deren Gemische und gegebenenfalls unter den syn- oder anti-Formen und ihren Gemischen.

2. Oxacephalosporinderivat gemäß Anspruch 1, dadurch gekennzeichnet, daß $R_1$ und R'' wie in a) in Anspruch 1 definiert sind, $R_4$ einen Rest der allgemeinen Formel (II)

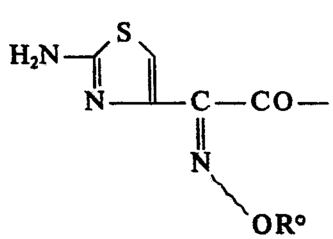 (II)

bedeutet,
worin R° wie in Anspruch 1 definiert ist und dessen Aminfunktion vorher geschützt wurde durch eine t-Butoxycarbonyl-, 2,2,2-Trichloräthoxycarbonyl-, Trichloracethyl-, Chloracethyl-, Trithyl-, Dibenzyl-, Benzyl-, Benzyloxycarbonyl-, p-Nitrobenzyloxycarbonyl-, p-Methoxybenzyloxycarbonyl-, Formyl- oder Trifluoracethylgruppe und $R_2$ wie in Anspruch 1 definiert ist.

3. Oxacephalosporinderivat gemäß Anspruch 1, dadurch gekennzeichnet, daß $R_1$ und R'' wie in a) in Anspruch 1 definiert sind, $R_4$ einen Rest der allgemeinen Formel (II)

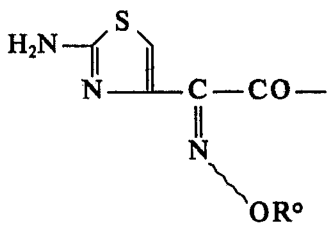

(II)

bedeutet,
worin die Aminfunktion frei oder geschützt ist und worin R° eine Trityl-, Tetrahydropyranyl- oder 2-Methoxy-2-propyl-Schutzgruppe für die Oximfunktion bedeutet und $R_2$ wie in Anspruch 1 definiert ist.

4. Oxacephalosporinderivat gemäß Anspruch 1, dadurch gekennzeichnet, daß $R_1$ und R″ wie in a) in Anspruch 1 definiert sind und $R_4$ einen Rest der allgemeinen Formel (II)

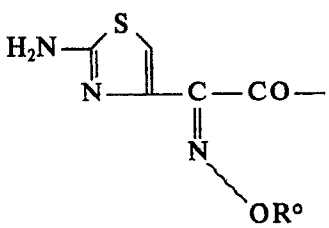

(II)

bedeutet,
dessen Aminfunktion frei oder geschützt ist und worin R° eine Carboxyalkylgruppe [der allgemeinen Formel (III)

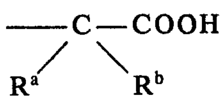

(III)

worin $R^a$ und $R^b$ wie in Anspruch 1 definiert sind] bedeutet, die durch einen Rest, ausgewählt unter Methoxymethyl, t-Butyl, Benzhydryl, Benzyl, Nitrobenzyl und p-Methoxybenzyl, geschützt ist und $R_2$ wie in Anspruch 1 definiert ist.

5. Oxacephalosporinderivat gemäß Anspruch 1, dadurch gekennzeichnet, daß $R_1$ und R″ wie in a) in Anspruch 1 definiert sind, $R_4$ einen $\alpha$-Carboxyarylacetylrest bedeutet, dessen Carboxygruppe durch einen Rest geschützt ist, ausgewählt unter Methoxymethyl, t-But/l, Benzhydryl, Benzyl, Nitrobenzyl und p-Methoxybenzyl.

6. Oxacephalosporinderivat gemäß Anspruch 1, dadurch gekennzeichnet, daß $R_2$, $R_4$ und R″ wie in Anspruch 1 definiert sind, der leicht entfernbare Rest, dargestellt durch $R_1$, ausgewählt ist unter den Methoxymethyl-, t-Butyl-, Benzhydryl-, Benzyl-, p-Nitrobenzyl- und p-Methoxybenzylresten.

7. Oxacephalosporinderivat gemäß Anspruch 1, dadurch gekennzeichnet, daß $R_1$ und R″ wie in b) in Anspruch 1 definiert sind, $R_4$ einen leicht entfernbaren Rest bedeutet, ausgewählt unter:

1. Benzhydryl oder Trityl,
2. einem Acylrest der allgemeinen Formel (V)

$R_5CO-$ (V)

worin $R_5$ bedeutet:

a) ein Wasserstoffatom, einen Alkylrest mit 1 bis 7 Kohlenstoffatomen, Methyl substituiert durch 1 bis 3 Halogenatome, Alkenyl mit 3 bis 7 Kohlenstoffatomen, oder Cyanomethyl,
b) einen Phenylrest (der bis zu dreimal durch ein Halogenatom oder durch Hydroxy-, Nitro-, Cyano-, Trifluormethyl-, Alkyl- oder Alkyloxyreste substituiert sein kann) oder einen Thienyl - 2- oder -3-Rest,
c) einen Rest der allgemeinen Formel (VIa)

$R'_5YCH_2-$ (VIa)

worin $R'_5$ einen in b) definierten Rest bedeutet und Y ein Schwefel- oder Sauerstoffatom bedeutet,

d) einen Arylalkylrest der allgemeinen Formel (VIb)

$R''_5CH_2-$ (VIb)

worin R″$_5$ einen Phenylrest bedeutet (der bis zu dreimal durch Hydroxy-, Alkyl- oder Alkylox yreste substituiert sein kann) oder einen heterocyclischen Rest, ausgewählt unter Thienyl-2 oder -3, Furyl-2 oder -3 oder Tetrazolyl-1,

3. einem Rest der allgemeinen Formel (VII)

$R_6O\,CO-$ (VII)

worin $R_6$ einen verzweigten, nicht substituierten Alkylrest, einen geraden oder verzweigten Alkylrest mit einem oder mehreren Substituenten, ausgewählt unter den Halogenatomen oder Cyano-, Phenyl- oder substituierten Phenylresten (durch ein oder mehrere Halogenatome oder Alkyl-, Alkyloxy-, Nitro- oder Phenylreste), einen 2-Trimethylsilyl-äthylrest, einen Vinyl- oder Allylrest oder einen Chinolylrest bedeutet,

4. einem Rest der allgemeinen Formel (VIII) oder (IX)

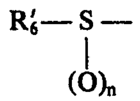 (VIII) oder $R'_6$—Se— (IX)

worin das Symbol $R'_6$ einen Alkyl- oder Phenylrest oder Phenyl substituiert durch ein oder mehrere Halogenatome oder Nitro- oder Alkylreste bedeutet und n gleich 0 oder 1 ist,

5. oder auch $R_4$ bedeutet einen bis-(4-Nitrobenzyl)-phosphorylrest,

6. oder auch $R_4NH$ durch einen Alkylaminomethylenamino-Rest oder durch einen Rest der allgemeinen Formel (X)

$Ar-CH=N-$ (X)

worin Ar eine Phenylgruppe, gegebenenfalls substituiert durch einen oder mehrere Reste, ausgewählt unten Hydroxy, Nitro, Alkyl- oder Alkyloxy, ersetzt ist.

8. Verfahren zur Herstellung eines Produkts gemäß Anspruch 1, worin $R_1$, $R_2$ $R_4$ und R″ wie in Anspruch 1 definiert sind, mit Ausnahme der Bedeutung eines Wasserstoffatoms für $R_4$, dadurch gekennzeichnet, daß man entweder ein aktiviertes Derivat der Säuren $R_3SO_3H$ und $R'_3COOH$, ausgewählt unter (XI)

$(R_3SO_2)_2O$
$R_3SO_2Hal$
$(R'_3CO)_2O$
und $R'_3COHal$ (XI)

($R_3$ und $R'_3$ sind wie in Anspruch 1 definiert und Hal bedeutet ein Halogenatom) oder ein Halogenierungsmittel auf ein Produkt der allgemeinen Formel (XII)

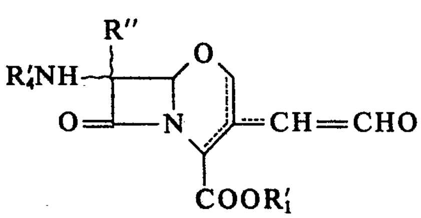 (XII)

(oder auf ein Gemisch von dessen Isomeren) einwirken läßt, das in Bicycloocten-2- oder -3-Form oder 3-Oxoäthyliden-bicyclooctan-Form vorliegt und worin $R'_1$ ein leicht entfernbarer Schutzrest ist und entweder $R'_4$ wie $R_4$ in Anspruch 1 in a) definiert ist mit der Ausnahme der Bedeutung Wasserstoff, wobei falls $R'_4$ einen Aminorest enthält, dieser letztere geschützt ist, und R″ ist ein Wasserstoffatom oder ein Methoxyrest in $7\alpha$-Stellung oder
$R'_4$ ist wie $R_4$ in Anspruch 1 in b) definiert und R″ ist ein Wasserstoffatom oder ein Methoxyrest in $7\alpha$-Stellung oder ein Wasserstoffatom in $7\beta$-Stellung,
und daß man dann gegebenenfalls die Schutzreste entfernt und die Isomeren des erhaltenen Produkts gegebenenfalls trennt.

9. Verfahren zur Herstellung eines Produkts gemäß Anspruch 1, worin $R_1$, $R_2$ und R″ wie in Anspruch 1 in a) definiert sind und $R_4$ ein Wasserstoffatom ist, dadurch gekennzeichnet, daß man den

Rest $R_4$ entfernt oder daß man gegebenenfalls gleichzeitig den Rest $R_4$ und den Rest $R_1$ eines Produkts gemäß Anspruch 1 entfernt, worin $R_1$ und $R_4$ wie in Anspruch 1 in b) definiert sind und daß man dann gegebenenfalls das erhaltene Produkt in seine Isomeren auftrennt.

10. Verfahren zur Herstellung eines Produkts gemäß Anspruch 1, worin $R_1$, $R_2$, $R_4$ und R″ wie in Anspruch 1 in a) definiert sind, mit Ausnahme der Bedeutung eines Wasserstoffatoms für $R_4$, dadurch gekennzeichnet, daß man mittels einer Säure, dargestellt durch die allgemeine Formel (XXI)

$$R_4-OH \qquad \text{(XXI)}$$

(worin $R_4$ wie oben definiert ist, und deren Aminfunktion gegebenenfalls vorher geschützt wurde) oder eines ihrer reaktiven Derivate ein Oxacephalosporin gemäß Anspruch 1, worin $R_4$ ein Wasserstoffatom bedeutet, acyliert, und daß man dann gegebenenfalls die Schutzgruppen entfernt und daß man das erhaltene Produkt gegebenenfalls in seine Isomeren auftrennt.

**Patentanspruch für den Vertragsstaat: AT**

Verfahren zur Herstellung eines Oxacephalosporinderivats der allgemeinen Formel (I)

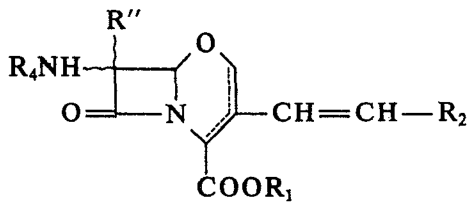

(I)

das in Bicycloocten-2- oder -3-Form vorliegt und dessen Substituent in 3-Stellung die E- oder Z-Stereoisomerie aufweist, und worin

a) das Symbol $R_1$ ein Wasserstoffatom oder einen leicht entfernbaren Säureschutzrest bedeutet, das Symbol $R_4$ ein Wasserstoffatom oder einen Rest der allgemeinen Formel (II)

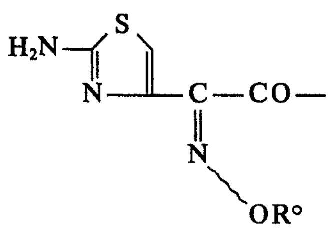

(II)

bedeutet,
[dessen Aminfunktion frei oder geschützt ist und worin R° ein Wasserstoffatom, einen Oxim-Schutzrest, einen Alkyl-, Vinyl- oder Carboxyalkylrest, frei oder geschützt, dargestellt durch die allgemeine Formel (III)

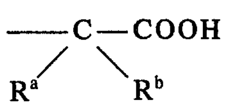

(III)

bedeutet,
worin die Reste $R^a$ und $R^b$, welche identisch oder verschieden sind, Wasserstoffatome oder Alkylreste bedeuten oder zusammen einen Alkylenrest mit 2 oder 3 Kohlenstoffatomen bilden]
oder
$R_4$ bedeutet einen $\alpha$-Carboxyarylacetylrest, dessen Carboxygruppe frei oder geschützt sein kann und worin Aryl Phenyl (gegebenenfalls substituiert durch einen freien oder geschützten p-Hydroxyrest) oder Thienyl-2 oder -3 bedeutet, und
das Symbol R″ ein Wasserstoffatom oder einen Methoxyrest in $7\alpha$-Stellung bedeutet, oder

b) das Symbol $R_1$ einen leicht entfernbaren Säureschutzrest bedeutet,
das Symbol $R_4$ einen leicht entfernbaren Rest bedeutet, und
das Symbol R″ein Wasserstoffatom oder einen Methoxyrest in $7\alpha$ oder ein Wasserstoffatom in $7\beta$ bedeutet, und
das Symbol $R_2$ einen Rest der allgemeinen Formel (IVa) oder (IVb)

$$-O-SO_2R_3 \qquad \text{(IVa)}$$

oder

$-O-COR'_3$ (IVb)

[worin $R_3$ ein gerader oder verzweigter Alkylrest mit 1 bis 4 Kohlenstoffatomen, Trifluormethyl, Trichlormethyl oder Phenyl, nicht substituiert oder substituiert durch ein Halogenatom oder durch einen Alkyl- oder Nitrorest, ist und
$R'_3$ wie $R_3$ definiert ist oder einen Acylmethyl-, 2-Acyläthyl-, 2-Acylpropyl-, Alkyloxycarbonylmethyl-, 2-Alkyloxycarbonyläthyl- oder 2-Alkyloxycarbonyl-propyl-Rest bedeutet], oder ein Halogenatom bedeutet,
wobei die oben erwähnten Alkyl- oder Acylreste und -teile (wenn nicht anderes angegeben ist) gerade oder verzweigt sind und 1 bis 4 Kohlenstoffatome enthalten,
unter seinen E- oder Z-Formen und von deren Gemischen und gegebenenfalls unter seinen syn- oder anti-Formen und deren Gemischen, dadurch gekennzeichnet, daß man

A. zur Herstellung eines Produkts, in dessen Formel $R_4$ anders als Wasserstoff ist, entweder ein aktiviertes Derivat der Säuren $R_3SO_3H$ und $R'_3COOH$, ausgewählt unter (XI)

$(R_3SO_2)_2O$
$R_3SO_2Hal$
$(R'_3CO)_2O$
und $R'_3COHal$ (XI)

($R_3$ und $R'_3$ sind wie oben definiert und Hal bedeutet ein Halogenatom) oder ein Halogenierungsmittel, auf ein Produkt der allgemeinen Formel (XII)

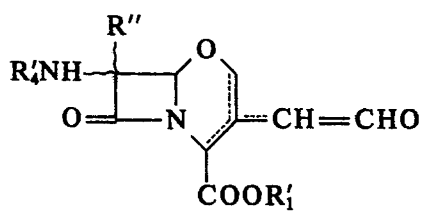
(XII)

(oder auf ein Gemisch seiner Isomeren) einwirken läßt, das in Bicycloocten-2- oder-3-Form oder in 3-Oxoäthyliden-bicyclooctan-Form vorliegt und worin $R'_1$ einen leicht entfernbaren Schutzrest bedeutet und entweder $R'_4$ wie $R_4$ oben in a) definiert ist, mit Ausnahme der Bedeutung Wasserstoff, wobei falls $R'_4$ einen Aminrest enthält, dieser letztere geschützt ist, und R″ ein Wasserstoffatom oder einen Methoxyrest in $7\alpha$ bedeutet, oder $R'_4$ ist wie $R_4$ in b) definiert und R″ bedeutet ein Wasserstoffatom oder einen Methoxyrest in $7\alpha$-Stellung oder ein Wasserstoffatom in $7\beta$-Stellung, und daß man dann gegebenenfalls die Schutzgruppen entfernt und gegebenenfalls die Isomeren des erhaltenen Produkts auftrennt, und daß man dann

B. zur Herstellung eines Produkts, in dessen Formel $R_4$ ein Wasserstoffatom bedeutet, den Rest $R_4$ entfernt oder gegebenenfalls gleichzeitig den Rest $R_4$ und den Rest $R_1$ eines Produkts, hergestellt, gemäß A) und worin $R_1$ und $R_4$ wie oben in b) definiert sind, entfernt, und daß man dann gegebenenfalls das erhaltene Produkt in seine Isomeren auftrennt, und dann

C. zur Herstellung eines Produkts, in dessen Formel $R_4$ anders als ein Wasserstoffatom ist, mittels einer Säure, dargestellt durch die allgemeine Formel (XXI)

$R_4-OH$ (XXI)

(worin $R_4$ wie oben definiert ist und wobei gegebenenfalls die Aminfunktion vorher geschützt wurde) oder eines ihrer reaktiven Derivate ein Oxacephalosporin, hergestellt gemäß B), acyliert und daß man dann gegebenenfalls die Schutzgruppen entfernt und gegebenenfalls das erhaltene Produkt in seine Isomeren auftrennt.

**Claims for the Contracting States: BE, CH, DE, FR, GB, IT, LI, LU, NL, SE**

1. Oxacephalosporin derivative, characterised in that it corresponds to the general formula:

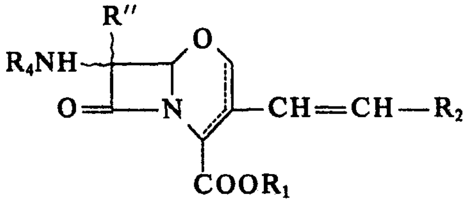

(I)

which is in the form of a bicyclooct-2-ene or bicyclooct-3-ene, in which the substituent in the 3-position exhibits E or Z stereoisomerism and in which

a) the symbol $R_1$ represents a hydrogen atom or an acidprotecting radical which can easily be removed, the symbol $R_4$ represents a hydrogen atom or a radical of the general formula:

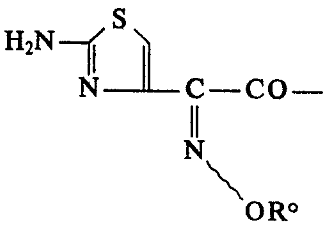

(II)

[in which the amine group is free or protected and in which R° represents a hydrogen atom, an oxime-protecting radical, an alkyl radical, a vinyl radical or a free or pretected carboxalkyl radical represented by the general formula:

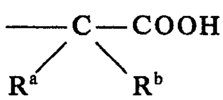

(III)

in which the radicals $R^a$ and $R^b$, which are identical or different represent hydrogen atoms or alkyl radicals or together form an alkylen radical containing 2 or 3 carbon atoms], or $R_4$ represents an $\alpha$-carboxyarylacetyl radical, in which the carboxyl group can be free or pretected and in which aryl represents phenyl (optionally substituted by a free or protected p-hydroxyl radical), thien-2-yl or thien-3-yl, and the symbol R″ represents a hydrogen atom or a methoxy radical in the $7\alpha$-position, or alternatively

b) the symbol $R_1$ represents an acid-protecting radical which can easily be removed, the symbol $R_4$ represents a radical which can easily be removed and the symbol R″ represents a hydrogen atom or a methoxy radical in the $7\alpha$-position or a hydrogen atom in the $7\beta$-position, and the symbol $R_2$ represents a radical of the general formula:

$$-O-SO_2-R_3 \quad \text{(IVa)}$$

or

$$-O-COR'_3 \quad \text{(IVb)}$$

[in which formulae $R_3$ is a linear or brached alkyl radical containing 1 to 4 carbon atoms, a trifluoromethyl radical, a trichloromethyl radical or a phenyl radical which is unsubstituted or substituted by a halogen atom or by an alkyl or nitro radical, and $R'_3$ is defined in the same way as $R_3$ or represents an acylmethyl, 2-acylethyl, 2-acylpropyl, alkoxycarbonylmethyl, 2-alkoxycarbonylethyl or 2-alkoxycarbonylpropyl radical] or a halogen atom,
it being understood that the abovementioned alkyl or acyl radicals and portions are linear or branched (unless stated otherwise) and contain 1 to 4 carbon atoms,
in its E or Z forms and mixtures thereof, and, if appropriate, in its syn or anti forms and mixtures thereof,

2. Oxacephalosporin derivative according to claim 1, characterised in that, with $R_1$ and R″ defined as under a) in claim 1, $R_4$ represents a radical of the general formula:

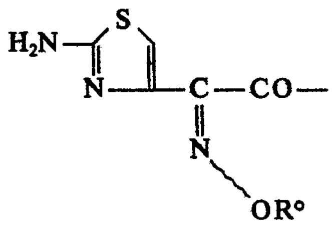

(II)

in which R° is defined according to claim 1, and in which the amino group is protected beforehand by a t-butoxycarbonyl, 2,2,2-trichloroethoxycarbonyl, trichloroacetyl, chloroacetyl, trityl, dibenzyl, benzyl, benzyloxycarbonyl, p-nitrobenzyloxycarbonyl, p-methoxybenzyloxycarbonyl, formyl or trifluoroacetyl group, and $R_2$ is defined as in claim 1.

3. Oxacephalosporin derivative according to claim 1, characterised in that, whit $R_1$ and R″ defined as under a) in claim 1, $R_4$ represents a radical of the general formula:

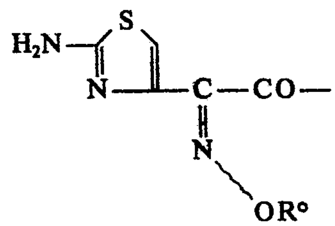

(II)

in which the amine group is free or protected and in which R° represents an trityl, tetrahydropyranyl or 2-methoxy-prop-2-yl group protecting the oxime group, and $R_2$ is defined as in claim 1.

4. Oxacephalosporin derivative according to claim 1, characterised in that, with $R_1$ and R″ defined as under a) in claim 1, $R_4$ represents a radical of the general formula:

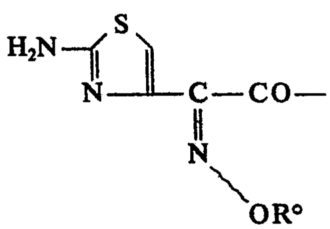

(II)

in which the amine group is free or protected and in which R° represents a carboxyalkyl group of the general formula:

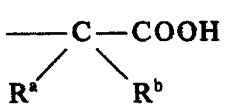

(III)

[in which $R^a$ and $R^b$ are defined as in claim 1], protected by a radical chosen from amongst methoxymethyl, t-butyl, benzhydryl, benzyl, nitrobenzyl and p-methoxybenz/l, and $R_2$ is defined as in claim 1.

5. Oxacephalosporin derivative according to claim 1, characterised in that, with $R_1$ and R″ defined as under a) in claim 1, $R_4$ represents an $\alpha$-carboxyarylacetyl radical in which the carboxyl group is protected by a radical chosen from amongst methoxymethyl, t-butyl, benzhydryl, benzyl, nitrobenzyl and p-methoxybenzyl.

6. Oxacephalosporin derivative according to claim 1, characterised in that, with $R_2$,$R_4$ and R″ defined as in claim 1, the radical which can easily be removed and which is represented by $R_1$ is chosen from amongst methoxymethyl, t-butyl, benzhydryl, benzyl, p-nitrobenzyl and p-methoxybenzyl radicals.

7. Oxacephalosporin derivative according to claim 1, characterised in that, with $R_1$ and R″ defined as under b) in claim 1, $R_4$ represents a radical which can easily be removed and which is chosen from amongst:

1. benzhydryl or trityl,
2. an acyl radical of the general formula:

$R_5CO-$ (V)

in which $R_5$ represents:

a) a hydrogen atom, an alkyl radical containing 1 to 7 carbon atoms, a methyl radical substituted by 1 to 3 halogen atoms, an alkenyl radical containing 3 to 7 carbon atoms or a cyanomethyl radical,
b) a phenyl radical (which can be up to trisubstituted by a halogen atom or by hydroxyl, nitro, cyano, trifluoromethyl, alkyl or alkoxy radicals) or a thien-2-yl or thien-3-yl radical,
c) a radical of the general formula:

$R'_5YCH_2-$ (VIa)

in which $R'_5$ represents a radical defined under b) and Y represents a sulphur or oxygen atom, or

d) an arylalkyl radical of the general formula:

$R''_5CH_2-$ (VIb)

in which $R''_5$ represents a phenyl radical (which can be up to trisubstituted by hydroxyl, alkyl or alkoxy radicals) or a heterocyclic radical chosen from amongst thien-2-yl or thien-3-yl, furan-2-yl or furan-3-yl, or tetrazol-1-yl,

3. a radical of the general formula:

$R_6OCO-$ (VII)

in which $R_6$ represents an unsubstituted branched alkyl radical, a linear or branched alkyl radical carrying one or more substituents chosen from amongst halogen atoms or cyano radicals, phenyl radicals or phenyl radicals substituted by one or more halogen atoms or alkyl alkoxy, nitro or phenyl radicals a 2-trimethylsilylethyl radical, a vinyl or allyl radical or a quinolyl radical,

4. a radical of the general formula:

$R'_6-\underset{(O)_n}{\overset{}{S}}-$ (VIII) or $R'_6-Se-$ (IX)

in which formulae the symbol $R'_6$ represents an alkyl or phenyl radical or a phenyl radical substituted by one or more halogen atoms or nitro or alkyl readicals, and n is equal to 0 or 1, and

5. a bis-(4-nitrobenzyl)-phosphoryl radical, or alternatively
6. $R_4NH$ ist replaced by an alkylaminomethyleneamino radical or by a radical of the general formula:

$Ar-CH=N-$ (X)

in which Ar represents a phenyl group optionally substituted by one or more radicals chosen from amongst hydroxyl, nitro, alkyl or alkoxy.

8. A process for the preparation of a product according to claim 1 in which $R_1$, $R_2$, $R_4$ and R″ are defined as in claim 1, except that $R_4$ cannot represent a hydrogen atom, characterised in that either an activated derivative of the acids $R_3SO_3H$ and $R'_3COOH$, chosen from amongst:

$(R_3SO_2)_2O$
$R_3SO_2Hal$
$(R'_3CO)_2O$
and $R'_3COHal$ (XI)

($R_3$ and $R'_3$ being defined as in claim 1 and Hal representing a halogen atom), or a halogenating agent, is reacted with a product of the general formula:

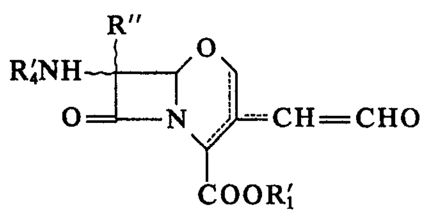

(XII)

(or with a mixture of its isomers), which is in the form of a bicyclooct-2-ene or bicyclooct-3-ene or a 3-oxoethylidenebicyclooctane and in which $R'_1$ is a pretecting radical which can easily be removed and either $R'_4$ is defined in the same way as $R_4$ in claim 1 under a), except that it cannot represent hydrogen, it being understood that if $R'_4$ contains an amino radical, the latter is protected, and R″ is a hydrogen atom or a methoxy radical in the 7α-position, or $R'_4$ is defined in the same way as $R_4$ in claim 1 under b) and R″ is a hydrogen atom or a methoxy radical in the 7α-position or a hydrogen atom in the 7β-position, and then, if appropriate, the protecting radicals are removed and, if appropriate, the isomers of the product obtaned are separated.

9. A process for the preparation of a product according to claim 1 in which $R_1$, $R_2$ and R″ are defined as in claim 1 under a) and $R_4$ is a hydrogen atom, characterised in that the radical $R_4$ is removed or, if appropriate, in that the radical $R_4$ and the radical $R_1$ are simultaneously removed from a product according to claim 1 in which $R_1$ and $R_4$ are defined as in claim 1 under b), and then, if appropriate, the product obtained is separated into its isomers.

10. A process for the preparation of a product according to claim 1 in which $R_1$, $R_2$, $R_4$ and R″ are defined as in claim 1 under a) except that $R_4$ cannot represent a hydrogen atom, characterised in that an oxacephalosporin according to claim 1 in which $R_4$ represents a hydrogen atom is acylated by means of an acid represented by the general formula:

$$R_4-OH \qquad (XXI)$$

(in which $R_4$ is defined as above and in which, if appropriate, the amine group is protected beforehand) or by means of one of its reactive derivatives, and then, if appropriate, the protecting radicals are removed and, if appropriate, the product obtained is separated into its isomers.

**Claim for the Contracting State: AT**

Process for the preparation of an oxacephalosporin derivative of the general formula:

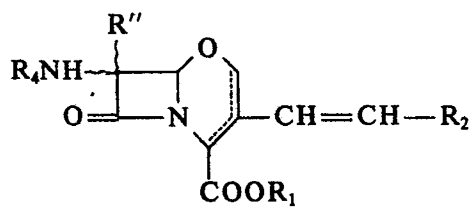

(I)

which is in the form of a bicycooct-2-ene or bicyclooct-3-ene, in which the substituent in the 3-position exhibits E or Z stereoisomerism and in which

a) the symbol $R_1$ represents a hydrogen atom or an acidprotecting radical which can easily be removed, the symbol $R_4$ represents a hydrogen atom or a radical of the general formula:

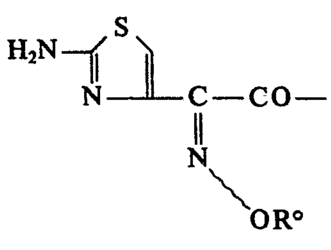

(II)

[in which the amine group is free or protected and in which R° represents a hydrogen atom, an oxime-protecting radical, an alkyl radical, a vinyl radical or a free or protected carboxyalkyl radical represented by the general formula:

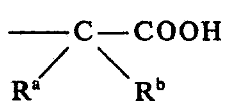

(III)

in which the radicals $R^a$ and $R^b$, which are identical or different, represent hydrogen atoms or alkyl radicals or together form an alkylene radical containing 2 or 3 carbon atoms], or $R_4$ represents an α-carboxyarylacetyl radical, in which the carboxyl group can be free or pretected and in which aryl represents phenyl (optionally substituted by a free or protected p-hydroxyl radical), thien-2-yl or thien-3-yl, and the symbol R″ represents a hydrogen atom or a methoxy radical in the 7α-position, or alternatively

b) the symbol $R_1$ represents an acid-protecting radical which can easily be removed, the symbol $R_4$ represents a radical which can easily be removed and the symbol R″ represents a hydrogen atom or a methoxy radical in the 7$\alpha$-position or a hydrogen atom in the 7$\beta$-position, and the symbol $R_2$ represents a radical of the general formula:

$-O-SO_2-R_3$ (IVa)

or

$-O-COR'_3$ (IVb)

[in which formulae $R_3$ is a linear or branched alkyl radical containing 1 to 4 carbon atoms, a trifluoromethyl radical, a trichloromethyl radical or a phenyl radical which is unsubstituted or substituted by a halogen atom or by an alkyl or nitro radical, and $R'_3$ is defined in the same way as $R_3$ or represents an acylmethyl, 2-acylethyl, 2-acylpropyl, alkoxycarbonylmethyl, 2-alkoxycarbonylethyl or 2-alkoxycarbonylpropyl radical] or a halogen atom,
it being understood that the abovementioned alkyl or acyl radicals and portions are linear or branched (unless stated otherwise) and contain 1 to 4 carbon atoms,
in its E or Z forms and mixtures thereof, and, if appropriate, in its syn or anti forms and mixtures thereof, characterised in that

A — for the preparation of a produkt in the formula of which $R_4$ is other than a hydrogen atom, either an activated derivative of the acids $R_3SO_3H$ and $R'_3COOH$, chosen from amongst:

$(R_3SO_2)_2O$
$R_3SO_2Hal$
$(R'_3CO)_2O$
and $R'_3COHal$ (XI)

($R_3$ and $R'_3$ being defined as above and Hal representing a halogen atom), or a halogenating agent, is reacted with a product of the general formula:

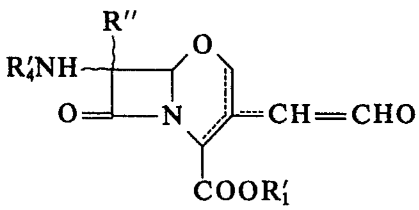

(XII)

(or with a mixture of its isomers), which is in the form of a bicyclooct-2-ene or bicyclooct-3-ene or a 3-oxoethylidenebicyclooctane and in which $R'_1$ represents a protecting radical which can easily be removed and either $R'_4$ is defined in the same way as $R_4$ above under a), except that it cannot represent hydrogen, it being understood that if $R'_4$ contains an amino radical, the latter is protected, and R″ represents a hydrogen atom or a methoxy radical in the 7$\alpha$-position, or $R'_4$ is defined in the same way as $R_4$ under b) and R″ represents a hydrogen atom or a methoxy radical in the 7$\alpha$-position or a hydrogen atom in the 7$\beta$-position, an then, if appropriate, the protecting radicals are removed and, if appropriate, the isomers of the product obtained are separated, then

B — for the preparation of a product in the formula of which $R_4$ represents a hydrogen atom, the radical $R_4$ is removed or, if appropriate, the radical $R_4$ and the radical $R_1$ are simultaneously removed from a product prepared according to A- in which $R_1$ and $R_4$ are defined as above under b), and then, if appropriate, the product obtained is separated into its isomers, and then,

C — for the preparation of a product in the formula of which $R_4$ is other than a hydrogen atom, an oxacephalosporin prepared according to B- is acylated by means of an acid represented by the general formula:

$R_4-OH$ (XXI)

(in which $R_4$ is defined as above and in which, if appropriate, the amine group is protected beforehand) or by means of one of its reactive derivatives, and then, if appropriate, the protecting radicals are removed, and if appropriate, the product obtained is separated into its isomers.